# EUROPEAN PATENT APPLICATION

(11) **EP 4 483 877 A1**
(43) Date of publication of application: **01.01.2025**
(21) Application number: 23759269.6
(22) Date of filing: 24.02.2023
(51) Int. Cl.: A61K 31/47, A61K 31/4406, A61K 39/00, A61K 39/395, C07K 16/28, C07K 16/46, A61P 35/00

(54) **PHARMACEUTICAL COMPOSITION COMPRISING ANTI-CTLA4-ANTI-PD-1 BISPECIFIC ANTIBODY AND CHIAURANIB**

(30) Priority: 24.02.2022 CN 202210174665
(71) Applicant: Akeso Pharmaceuticals, Inc., Guangzhou, Guangdong 510799 (CN); Shenzhen Chipscreen Biosciences Co., Ltd., Guangdong 518057 (CN)
(72) Inventor: XIA, Yu, Zhongshan, Guangdong 528437 (CN); LU, Xianping, Shenzhen, Guangdong 518057 (CN); LI, Baiyong, Zhongshan, Guangdong 528437 (CN); NING, Zhiqiang, Shenzhen, Guangdong 518057 (CN); WANG, Zhongmin, Zhongshan, Guangdong 528437 (CN); PAN, Desi, Shenzhen, Guangdong 518057 (CN)
(74) Representative: Cabinet Nony
(86) International application number: PCT/CN2023/078103
(87) International publication number: WO 2023/160647

(57) **Abstract**

The present invention belongs to the fields of tumor treatment and molecular immunology, and relates to a pharmaceutical composition comprising an anti-CTLA4-anti-PD-1 bispecific antibody and chiauranib. Specifically, the pharmaceutical composition comprises chiauranib or a pharmaceutically acceptable salt thereof or a crystalline form thereof, and at least one anti-CTLA4-anti-PD-1 bispecific antibody. The present invention also relates to use of the pharmaceutical composition.

## Description

### TECHNICAL FIELD

The present invention belongs to the field of tumor treatment and molecular immunology, and relates to a therapeutic combination comprising an anti-CTLA4-anti-PD-1 bispecific antibody and chiauranib and use thereof. Specifically, the present invention relates to a therapeutic combination of an anti-CTLA4-anti-PD-1 bispecific antibody comprising a mutation and chiauranib or a pharmaceutically acceptable salt thereof or a crystalline form thereof and use thereof.

### BACKGROUND

The transmembrane receptor PD-1 (programmed cell death protein-1) is a member of the CD28 gene family, and is expressed in activated T cells, B cells, and myeloid cells. Ligands of PD-1, both PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, play a role in killing tumor cells, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is the *in-vivo* injection of an anti-PD-1 antibody to modulate the expression of PD-1. Due to the broad-spectrum anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Homet M. B., Parisi G., et al., 2015, Semin Oncol., 42(3): 466-473), leukemia and anemia (Held SA, Heine A, et al., 2013, Curr Cancer Drug Targets., 13(7): 768-74).

Cytotoxic T lymphocyte-associated antigen 4 (CTLA4) and CD28 molecules are very similar in aspects of gene structure, chromosome location, sequence homology and gene expression. Both molecules are receptors of co-stimulatory molecule B7, and mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5).

Interleukin 2 (IL-2) is produced by T cells. It is a growth factor that regulates T cell subgroups, and an important factor in regulating immune responses. It promotes the proliferation of activated B cells, and participates in antibody responses, hematopoiesis and tumor surveillance. Recombinant human IL-2 has been approved by the U.S. FDA for treating malignant tumors, including melanoma, renal tumors, etc., while a clinical study is currently ongoing for treating chronic viral infections (Chavez, A.R., et al., 2009, Ann. N. Y. Acad. Sci., 1182:p.14-27). CTLA4 and CTLA4 antibodies are important influencing factors of T cell functions and interfere with the immune microenvironment in the body. *In-vitro* and *in-vivo* studies demonstrated that CTLA4 antibodies can specifically relieve the immunosuppression of CTLA4, activate T cells, and induce IL-2 generation, and are promising in wide applications in gene therapy against diseases such as tumors and parasite infections.

CTLA4 antibodies can produce a specific therapeutic effect on diseases and show remarkable efficacy, and may be used for supplementing traditional medicines and for exploring new means of gene therapy.

Bispecific antibodies, also known as bifunctional antibodies, are specific drugs that target two different antigens simultaneously, and can be purified and produced by immunomagnetic separation. Alternatively, they can be obtained by genetic engineering. The genetic engineering has corresponding flexibility in aspects of binding site optimization, synthetic form consideration, yield and the like, thus having certain advantages. Currently, over 45 existence forms have been demonstrated (Dafne Muller, Kontermann R E., 2010, BioDrugs, 24(2): 89-98). A number of developed bispecific antibodies are in the form of IgG-scFv, i.e., the Morrison format (Coloma MJ, Morrison SL., 1997, Nat Biotechnol., 15: 159-163), which has been demonstrated to be one of the ideal forms for the bifunctional antibodies because of its similarity to the naturally existing IgG forms and advantages in antibody engineering, expression and purification (Miller BR, Demarest SJ, et al., 2010, Protein Eng Des Sel, 23: 549-57; Fitzgerald J, Lugovskoy A., 2011, MAbs, 3: 299-309). ADCC (antibody-dependent cell-mediated cytotoxicity) refers to direct killing of a target cell by a killer cell (NK cell, macrophage, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) means that the specific binding of an antibody to a corresponding antigen on a cell membrane surface forms a complex and activates the complement system, which further forms an MAC on the surface of the target cell resulting in subsequent target cell lysis. Complements may cause cell lysis of various bacteria and other pathogenic organisms, and are an important defense mechanism against pathogenic organism infections.

Fc receptors belong to an immunoglobulin family that are expressed on the surfaces of specific immune cells to recognize antibody Fc regions and mediate immune responses. After the Fab region of the antibody recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcyR, FcaR and FcεR. FcyR can be further classified into four subtypes, FcyRI (CD64), FcyRII (CD32), FcyRIII (CD16) and FcRn (neonatal Fc receptor). Among these, FcyRI, FcyRII and FcyRIII are closely associated with ADCC effects. FcyRIII is the most predominant molecule mediating ADCC, with two highly homologous subtypes, FcyRIIIa and FcyRIIIb, in different cell types. In FcyRIIIa populations, two subtypes distinguished by sites of single-nucleotide polymorphism (SNP), FcyRIIIa_V158 with high affinity and FcγRIIIa_F158 with low affinity, are present. FcyRI has higher affinity for the Fc region of IgG and participates in ADCC process; FcyRII comprises three subtypes, FcyRIIa, FcyRIIb and FcyRIIc (also referred to as CD32a, CD32b and CD32c, respectively), among which FcyRIIa has ADCC activity; for FcyRIIa, two subtypes, FcγRIIa_H131 and FcγRIIa_R131, are present in humans due to single nucleotide mutation; FcyRIIb is an inhibitory receptor, and is a typical inhibitory FcyR that inhibits nearby ITAM pathways. For example, after the binding of the immune complex to BCR, the Fc fragment binds to FcyRIIb on the same cell, negatively regulating B cell activation and decreasing secretion of antibodies and cytokines (Hogarth PM, Pietersz GA., 2012, NATURE REVIEWS DRUG DISCOVERY, 11(4):311-331).

The IgG family comprises four members, IgG1, IgG2, IgG3 and IgG4, which differ in amino acids in the fragment crystallizable (Fc) region of the heavy chain constant region, resulting in their varying affinities for FcyRs. IgG1 is the most abundant subtype in humans and is also the most commonly used subtype in monoclonal antibody medication. IgG1 is capable of binding various FcyRs and is able to induce ADCC and CDC effects. IgG2 has the lowest affinity for FcyRs, but is still able to induce monocyte-mediated ADCC by binding to FcyRIIa. IgG3 features the highest binding capacity to FcyRs, and can induce ADCC and a greater CDC effect than IgG1. IgG4 molecules demonstrate a weak binding to FcyRs other than FcyRI, having a lower probability of causing CDC and NK cell-mediated ADCC. However, antibodies of the IgG4 subtype can mediate ADCP effects through binding to FcyRI, and the ADCP effects, present in antibody drugs targeting immune cells, may cause damage to immune cells, posing pharmacological adverse effects. At present, there is still a need for developing a novel anti-CTLA4-anti-PD-1 bispecific antibody to reduce or eliminate the damage caused by antibody-mediated ADCC, ADCP and/or CDC activity on immune cells to which the anti-CTLA4-anti-PD-1 bispecific antibody binds, and to improve the efficacy of the antibody drug.

Chemotherapeutic drugs are currently mainly classified into the following nine classes (He Jie, et al., Clinical Oncology, Beijing, People's Medical Publishing House, 2016: 230-237). The first class is drugs that directly bind to DNA and prevent DNA replication, including various alkylating agents, mitomycin, bleomycin, dacarbazine, platinum-based drugs (e.g., cisplatin and carboplatin), camptothecins, and derivatives thereof. The second class are drugs for preventing nucleic acid biosynthesis, which mainly affect the enzyme system of tumor cells and block the synthesis of precursors of DNA and RNA, thereby inhibiting the formation of DNA or RNA, mainly including methotrexate, fluorouracil, 6-mercaptopurine, hydroxyurea and cytarabine; such drugs mainly act on cells in S phase, and are antimetabolite chemotherapeutic drugs and cycle-specific anticancer drugs. The third class is chemotherapeutic drugs which affect transcription through the pharmacological mechanism that the drugs are inserted into the DNA double helix to form non-covalent binding with the DNA double helix, interfering with the transcription of genetic information on DNA to the DNA-dependent mRNA and causing damaged template function and hindered transcription. The fourth class is those affecting tubulin and mitosis, mainly including botanical drugs such as vinca alkaloids, podophyllotoxins and taxanes. The fifth class is drugs affecting the function of ribosomes and blocking protein synthesis; representatives of such drugs are harringtonine botanical drugs, which inhibit the initiation of protein synthesis, decompose the ribosome and release new peptide chain, but do not block the binding of mRNA and tRNA to ribosomes; such drugs cause the reduction of nuclear DNA and cytoplasmic RNA and depolymerization of polysomes, and inhibit mitosis. The sixth class is drugs that affect the tumor cell membrane such as concanavalin (Con-A) and phytohemagglutinin (PHA); they can bind to glycoprotein receptors on the cell membrane, thereby affecting DNA synthesis in tumor cells and preventing tumor cell from dividing. The seventh class is drugs that induce apoptosis, such as arsenic trioxide. The eighth class is hormones that treat tumors by regulating the endocrine system, including estrogens, antiestrogens, progestogens, androgens, antiandrogens, corticosteroids, and anticorticosteroids (including dichlorodiphenyldichloroethane and aminoglutethimide). The ninth class is anticancer targeted therapies, including monoclonal antibodies, epidermal growth factor signaling inhibitors (e.g., targeted drugs against receptor tyrosine kinase pathway), ubiquitin-proteasome inhibitors, and angiogenesis inhibitors.

Chiauranib is a multi-target multi-pathway selective kinase inhibitor developed by Shenzhen Chipscreen Biosciences Co., Ltd. Chiauranib can selectively inhibit a plurality of kinase targets such as Aurora B, CSF1R and VEGFR/PDGFR/c-Kit, thereby inhibiting tumor cell proliferation, enhancing anti-tumor immunity, inhibiting tumor angiogenesis and realizing an anti-tumor efficacy of a multi-pathway mechanism. In the previous clinical trial, single-drug treatment of chiauranib for small cell lung cancer (SCLC) patients after failure of multi-line therapy achieved very positive efficacy compared with historical control data. Chinese Patent (publication No. CN 111728974 A) discloses the structure of a chiauranib compound, which is N-(2-aminophenyl)-6-(7-methoxyquinoline-4-oxy)-1-naphthamide. The structural formula is shown in formula I.

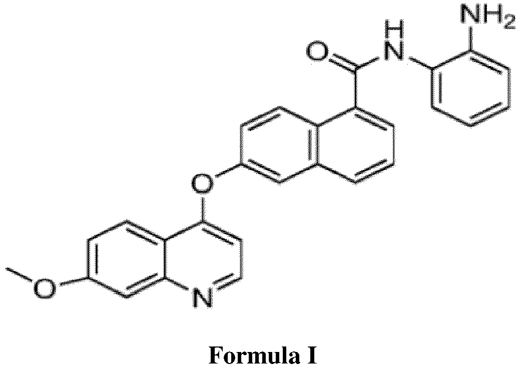

However, for a variety of tumors, the disease is still uncontrollable for a long term after chemotherapeutic drug treatment, and the 5-year survival rate is still very low. Therefore, developing a treatment mean or combination administration regimen with lower toxicity and higher efficacy is clinically of great meaning.

### SUMMARY

Through intensive studies and creative efforts, the inventors have creatively combined the anti-CTLA4/anti-PD-1 antibody and chiauranib properly, and the obtained therapeutic combination has a good anti-tumor effect.

The present invention is detailed below.

One aspect of the present invention relates to a therapeutic combination comprising chiauranib or a pharmaceutically acceptable salt thereof (e.g., hydrochloride salt) or a crystalline form thereof (e.g., non-solvated crystalline form A, B or C) and at least one (e.g., 1, 2 or 3) anti-CTLA4-anti-PD-1 bispecific antibody.

In some embodiments of the present invention, the therapeutic combination is provided, wherein the mass ratio of the chiauranib or the pharmaceutically acceptable salt thereof or the crystalline form thereof to the anti-CTLA4-anti-PD-1 bispecific antibody is 1:(1-1000), preferably 1:(5-500), and more preferably 1:(10-100).

In some embodiments of the present invention, the therapeutic combination is provided, wherein the mass ratio of the chiauranib or the pharmaceutically acceptable salt thereof or the crystalline form thereof to the anti-CTLA4-anti-PD-1 bispecific antibody is 1:(5-1000), preferably 1:(10-500), and more preferably 1:(10-100), 1:(10-150), 1:(10-200), 1:(10-250), 1:(10-300), 1:(10-350), 1:(10-400) or 1:(10-450).

In some embodiments of the present invention, the therapeutic combination is provided, wherein the anti-CTLA4-anti-PD-1 bispecific antibody is in a unit dose of 100 mg-1000 mg, 200 mg-800 mg, 200 mg-500 mg, 300 mg-600 mg, 400 mg-500 mg, or 450 mg.

In some embodiments of the present invention, the therapeutic combination is provided, wherein the chiauranib or the pharmaceutically acceptable salt thereof or the crystalline form thereof is in a unit dose of 0.1 mg-100 mg, 0.5 mg-50 mg, 1 mg-20 mg, 2 mg-15 mg, 3 mg-12 mg, 4 mg-8 mg, or 5 mg.

In some embodiments of the present invention, the therapeutic combination is provided, wherein
the therapeutic combination is a fixed combination, e.g., a pharmaceutical composition, or the therapeutic combination is a non-fixed combination, e.g., the chiauranib or the pharmaceutically acceptable salt thereof or the crystalline form thereof and the anti-CTLA4-anti-PD-1 bispecific antibody in the non-fixed combination are each present in a separate pharmaceutical composition.

In some embodiments of the present invention, the pharmaceutical composition is provided, wherein the pharmaceutical composition is independently a solid pharmaceutical composition or a liquid pharmaceutical composition.

In some embodiments of the present invention, the therapeutic combination is provided, wherein the pharmaceutical composition further comprises one or more pharmaceutically acceptable adjuvants, e.g., a carrier and/or an excipient.

In some embodiments of the present invention, the therapeutic combination is provided, wherein the pharmaceutical composition does not comprise an active pharmaceutical ingredient other than the chiauranib or the pharmaceutically acceptable salt thereof or the crystalline form thereof and the anti-CTLA4-anti-PD-1 bispecific antibody.

In some embodiments of the present invention, the therapeutic combination is provided, wherein the anti-CTLA4-anti-PD-1 bispecific antibody comprises:
a first protein functional region targeting PD-1 and
a second protein functional region targeting CTLA4,
wherein:
   the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
      or
   the first protein functional region is a single chain fragment variable, and the second protein functional region is an immunoglobulin, wherein a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively; a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively;
   the immunoglobulin is of human IgG1 subtype,
   and according to the EU numbering system, the heavy chain constant region of the immunoglobulin has mutations at any 2 or 3 of positions 234, 235 and 237, and the affinity constant of the bispecific antibody to FcyRIIIa and/or C1q is reduced after the mutation as compared to that before the mutation; preferably, the affinity constant is measured by a Fortebio Octet molecular interaction instrument.

In one or more embodiments of the present invention, the therapeutic combination is provided, wherein the anti-CTLA4-anti-PD-1 bispecific antibody is provided, wherein after the mutation described above, the affinity constant of the bispecific antibody to FcyRIIIa, FcyRI, FcyRIIa_H131, FcγRIIIa_V158 and/or FcyRIIb is reduced as compared to that before the mutation; preferably, the affinity constant is measured by a Fortebio Octet molecular interaction instrument.

In some embodiments of the present invention, the therapeutic combination is provided, wherein the anti-CTLA4-anti-PD-1 bispecific antibody is provided, wherein according to the EU numbering system, the heavy chain constant region of the immunoglobulin has the following mutations:
L234A and L235A; or
L234A and G237A; or
L235A and G237A;
   or
L234A, L235A and G237A.

In the present invention, letters before the position number represent amino acids before mutation, and letters after the position number represent amino acids after mutation, unless otherwise specified.

In some embodiments of the present invention, the therapeutic combination is provided, wherein the anti-CTLA4-anti-PD-1 bispecific antibody comprises:
a first protein functional region targeting PD-1 and
a second protein functional region targeting CTLA4,
wherein:
   the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
      or
   the first protein functional region is a single chain fragment variable, and the second protein functional region is an immunoglobulin, wherein a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively; a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively;
   the immunoglobulin is of human IgG1 subtype,
   according to the EU numbering system, the immunoglobulin comprises a heavy chain constant region having one of the following mutations:
      L234A and L235A; or
      L234A and G237A; or
      L235A and G237A; or
      L234A, L235A and G237A.

In some embodiments of the present invention, the therapeutic combination is provided, wherein the anti-CTLA4-anti-PD-1 bispecific antibody is provided, wherein according to the EU numbering system, the heavy chain constant region of the immunoglobulin further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present invention, the therapeutic combination is provided, wherein the anti-CTLA4-anti-PD-1 bispecific antibody is in the form of IgG-scFv, i.e., the Morrison format.

In some embodiments of the present invention, the therapeutic combination is provided, wherein the anti-CTLA4-anti-PD-1 bispecific antibody is provided, wherein
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16 and SEQ ID NO: 20; an amino acid sequence of the heavy chain variable region of the single chain fragment variable is selected from SEQ ID NO: 2, SEQ ID NO: 6, SEQ ID NO: 10, SEQ ID NO: 41 and SEQ ID NO: 43, and an amino acid sequence of the light chain variable region of the single chain fragment variable is selected from SEQ ID NO: 4, SEQ ID NO: 8, SEQ ID NO: 12, SEQ ID NO: 42 and SEQ ID NO: 44;
   or
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43, and an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain fragment variable is selected from SEQ ID NO: 14 and SEQ ID NO: 18, and an amino acid sequence of the light chain variable region of the single chain fragment variable is selected from SEQ ID NO: 16 and SEQ ID NO: 20.

In some embodiments of the present invention, the therapeutic combination is provided, wherein the anti-CTLA4-anti-PD-1 bispecific antibody is selected from any one of the following (1)-(20):
(1)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(2)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(3)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(4)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(5)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(6)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(7)
   an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(8)
   an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(9)
   an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(10)
   an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(11)
   an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(12)
   an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(13)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(14)
   an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(15)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(16)
   an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(17)
   an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(18)
   an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(19)
   an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
   and
(20)
   an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 18, and an amino acid sequence of the light chain variable region of the single chain fragment variable is set forth in SEQ ID NO: 20.

In some embodiments of the present invention, the therapeutic combination is provided, wherein the anti-CTLA4-anti-PD-1 bispecific antibody is provided:
an amino acid sequence of the heavy chain of the immunoglobulin is set forth in SEQ ID NO: 40, and an amino acid sequence of the light chain is set forth in SEQ ID NO: 24.

In some embodiments of the present invention, the therapeutic combination is provided, wherein the anti-CTLA4-anti-PD-1 bispecific antibody is provided, wherein the immunoglobulin or the antigen-binding fragment thereof binds to FcγRIIIa_F158, FcyRI, FcγRIIa_H131, FcγRIIIa_V158 and/or FcγRIIb with an affinity constant greater than about 10⁻⁷ M, for example, greater than about 10⁻⁶ M, 10⁻⁵ M, 10⁻⁴ M, or 10⁻³ M or greater; preferably, the affinity constant is measured by a Fortebio Octet molecular interaction instrument;
preferably, the immunoglobulin or the antigen-binding fragment thereof has no binding signal or a binding signal of less than 0.1 nm to FcγRIIIa_F158, FcyRI, FcγRIIa_H131, FcγRIIIa_V158 and/or FcyRIIb; preferably, the binding signal refers to a response value measured by a Fortebio Octet molecular interaction instrument.

In some embodiments of the present invention, the therapeutic combination is provided, wherein the anti-CTLA4-anti-PD-1 bispecific antibody is provided, wherein the immunoglobulin or the antigen-binding fragment thereof binds to C1q with an affinity constant greater than about 10⁻⁹ M, for example, greater than about 10⁻⁸ M, 10⁻⁷ M, 10⁻⁶ M, or 10⁻⁵ M or greater; preferably, the affinity constant is measured by a Fortebio Octet molecular interaction instrument;
preferably, the immunoglobulin or the antigen-binding fragment thereof has no binding signal or a binding signal of less than 0.1 nm to C1q; preferably, the binding signal refers to a response value measured by a Fortebio Octet molecular interaction instrument.

In some embodiments of the present invention, the therapeutic combination is provided, wherein the anti-CTLA4-anti-PD-1 bispecific antibody is provided, wherein the first protein functional region is linked to the second protein functional region either directly or via a linker fragment, and/or the heavy chain variable region of the single chain fragment variable is linked to the light chain variable region of the single chain fragment variable either directly or via a linker fragment.

In some embodiments of the present invention, the therapeutic combination is provided, wherein the anti-CTLA4-anti-PD-1 bispecific antibody is provided, wherein the linker fragment is (GGGGS)n, n being a positive integer; preferably, n is 1, 2, 3, 4, 5 or 6.

In some embodiments of the present invention, the therapeutic combination is provided, wherein the anti-CTLA4-anti-PD-1 bispecific antibody is provided, wherein the numbers of the first protein functional region and the second protein functional region are each independently 1, 2, or more.

In one or more embodiments of the present invention, the therapeutic combination is provided, wherein the anti-CTLA4-anti-PD-1 bispecific antibody is provided, wherein the number of the first protein functional region is 1 and the number of the second protein functional region is 2.

In one or more embodiments of the present invention, the therapeutic combination is provided, wherein the anti-CTLA4-anti-PD-1 bispecific antibody is provided, wherein the single chain fragment variable is linked to the C terminus of the heavy chain of the immunoglobulin. Since an immunoglobulin has two heavy chains, two single chain fragment variable molecules are linked to one immunoglobulin molecule. Preferably, the two single chain fragment variable molecules are identical. Preferably, the single chain fragment variable is linked to the C terminus of the heavy chain of the immunoglobulin by forming an amide bond via the aforementioned linker fragment.

In one or more embodiments of the present invention, the constant regions of the immunoglobulin are humanized. For example, the heavy chain constant region is Ig gamma-1 chain C region, ACCESSION: P01857, and the light chain constant region is Ig kappa chain C region, ACCESSION: P01834.

In one or more embodiments of the present invention, the therapeutic combination is provided, wherein the anti-CTLA4-anti-PD-1 bispecific antibody binds to CTLA4 protein and/or PD-1 protein with a K_{D} less than about 10⁻⁵ M, e.g., less than about 10⁻⁶ M, 10⁻⁷ M, 10⁻⁸ M, 10⁻⁹ M or 10⁻¹⁰ M or less.

In one or more embodiments of the present invention, the therapeutic combination is provided, wherein the anti-CTLA4-anti-PD-1 bispecific antibody is a monoclonal antibody. In one or more embodiments of the present invention, the therapeutic combination is provided, wherein the anti-CTLA4-anti-PD-1 bispecific antibody is a humanized antibody. In some embodiments of the present invention, the therapeutic combination is provided, wherein the anti-CTLA4-anti-PD-1 bispecific antibody is provided, wherein the single chain fragment variable is linked to the C terminus of the heavy chain of the immunoglobulin.

In some embodiments of the present invention, the therapeutic combination is provided, wherein the anti-CTLA4-anti-PD-1 bispecific antibody comprises:
a first protein functional region targeting PD-1 and
a second protein functional region targeting CTLA4,
the number of the first protein functional region is 1, and the number of the second protein functional region is 2;
wherein the first protein functional region is an immunoglobulin, and the second protein functional region is a single chain fragment variable;
an amino acid sequence of the heavy chain of the immunoglobulin is set forth in SEQ ID NO: 40, and an amino acid sequence of the light chain is set forth in SEQ ID NO: 24;
an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
the single chain fragment variable is linked to the C terminus of the heavy chain of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

Another aspect of the present invention relates to a kit product comprising the therapeutic combination according to any embodiment of the present invention, and a package insert. In some embodiments of the present invention, the kit product comprises a first product and a second product in separate packages,
wherein
the first product comprises the anti-CTLA4-anti-PD-1 bispecific antibody according to any embodiment of the present invention;
the second product comprises the chiauranib or the pharmaceutically acceptable salt thereof (e.g., hydrochloride salt) or the crystalline form thereof (e.g., non-solvated crystalline form A, B or C);
preferably, the first product and the second product further independently comprise one or more pharmaceutically acceptable adjuvants;
preferably, the kit product further comprises a package insert.

In one or more embodiments of the present invention, the kit product is provided, wherein the unit dose of the first product is 100 mg-1000 mg, 200 mg-800 mg, 200 mg-500 mg, 300 mg-600 mg, 400 mg-500 mg, or 450 mg, based on the mass of the anti-CTLA4-anti-PD-1 bispecific antibody.

In one or more embodiments of the present invention, the kit product is provided, wherein the unit dose of the second product is 0.1 mg-100 mg, 0.5 mg-50 mg, 0.5 mg-10 mg, 1 mg-10 mg, 2 mg-8 mg or 1 mg-5 mg, or 5 mg, based on the mass of the chiauranib or the pharmaceutically acceptable salt thereof or the crystalline form thereof.

In one or more embodiments of the present invention, the kit product is provided, wherein the unit dose of the second product is 1 mg-20 mg, 2 mg-15 mg, 3 mg-12 mg, 4 mg-8 mg, or 5 mg, based on the mass of the chiauranib or the pharmaceutically acceptable salt thereof or the crystalline form thereof.

Yet another aspect of the present invention relates to use of the therapeutic combination according to any embodiment of the present invention for preparing a drug for the treatment or prevention of a tumor;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

MSI refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L) and microsatellite stability (MSS). The major cause of MSI is DNA mismatch repair (MMR) deficiency. Human mismatch repair genes (MMR genes) can express corresponding mismatch repair proteins through transcription and translation. Expression absence of any MMR protein may lead to mismatch repair deficiency, and base pair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189: 45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient mismatch repair (pMMR). The detection of dMMR is to perform an immunohistochemical assay of protein expression for four mismatch genes of MSH2, MLH1, MSH6 and PMS2 based on tumor specimens (including surgical specimens and aspiration specimens). Expression absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for PCR assay, inconsistency in ≥30% loci is defined as MSI-H, consistency in all loci is defined as MSS, and inconsistency between 0 and 30% is defined as MSI-L.

The therapeutic combination according to any embodiment of the present invention for use in the treatment or prevention of a tumor;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

Yet another aspect of the present invention relates to a method for treating or preventing a tumor, comprising a step of administering to a subject in need an effective amount of the therapeutic combination according to any embodiment of the present invention;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
   colon cancer, rectal cancer, endometrial cancer, gastric cancer, mesothelioma, sarcoma, adrenocortical carcinoma, malignant melanoma and ovarian germ cell neoplasm.

In one or more embodiments of the present invention, the method for treating or preventing a tumor is provided, wherein the step of administrating to a subject in need an effective amount of the anti-CTLA4-anti-PD-1 bispecific antibody is before or after a surgical treatment and/or before or after a radiotherapy.

In one or more embodiments of the present invention, the method for treating or preventing a tumor is provided, wherein
the unit dose of the anti-CTLA4-anti-PD-1 bispecific antibody is 0.1-100 mg, preferably 1-10 mg (e.g., 1 mg, 2 mg, 3 mg, 4 mg, 5 mg, 6 mg, 7 mg, 8 mg, 9 mg or 10 mg) per kg body weight; alternatively, the unit dose of the anti-CTLA4-anti-PD-1 bispecific antibody is 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg or about 1000 mg), preferably 50-500 mg, 100-400 mg, 150-300 mg, 150-250 mg or 200 mg in each subject;
preferably, the dose is given once every 3 days, 4 days, 5 days, 6 days, 10 days, 1 week, 2 weeks, or 3 weeks;
preferably, the route of administration is intravenous drip infusion or intravenous injection. In some embodiments, the administration of the anti-CTLA4-anti-PD-1 bispecific antibody is performed in cycles of 2 weeks (14 days) or 3 weeks (21 days), and preferably, the anti-CTLA4-anti-PD-1 bispecific antibody is administered intravenously on the first day (D1) of each cycle. For example, the anti-CTLA4-anti-PD-1 bispecific antibody is administered once every two weeks (q2w) or three weeks (q3w).

In one or more embodiments of the present invention, the method for treating or preventing a tumor is provided, wherein the anti-CTLA4-anti-PD-1 bispecific antibody and chiauranib are administered simultaneously or sequentially.

Antibody drugs, especially monoclonal antibodies (mAbs), have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda Md).

The amino acid sequences of the CDR regions of the monoclonal antibody sequences in (1)-(13) below are analyzed by technical means well known to those skilled in the art, for example, by a VBASE2 database, and the results are as follows:

### (1) 14C12

An amino acid sequence of the heavy chain variable region is set forth in SEQ ID NO: 14, and an amino acid sequence of the light chain variable region is set forth in SEQ ID NO: 16. Amino acid sequences of the 3 CDR regions of the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)

Amino acid sequences of the 3 CDR regions of the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (2) 14C12H1L1

An amino acid sequence of the heavy chain variable region is set forth in SEQ ID NO: 18, and an amino acid sequence of the light chain variable region is set forth in SEQ ID NO: 20.

The amino acid sequences of the 3 CDR regions of the heavy chain variable region are identical to those of 14C12.

The amino acid sequences of the 3 CDR regions of the light chain variable region are identical to those of 14C12.

### (3) 4G10

An amino acid sequence of the heavy chain variable region is set forth in SEQ ID NO: 2, and an amino acid sequence of the light chain variable region is set forth in SEQ ID NO: 4. Amino acid sequences of the 3 CDR regions of the heavy chain variable region are as follows:
HCDR1: GYSFTGYT (SEQ ID NO: 33)
HCDR2: INPYNNIT (SEQ ID NO: 34)
HCDR3: ARLDYRSY (SEQ ID NO: 35)

Amino acid sequences of the 3 CDR regions of the light chain variable region are as follows:
LCDR1: TGAVTTSNF (SEQ ID NO: 36)
LCDR2: GTN (SEQ ID NO: 37)
LCDR3: ALWYSNHWV (SEQ ID NO: 38)

### (4) 4G10H1L1

An amino acid sequence of the heavy chain variable region is set forth in SEQ ID NO: 6, and an amino acid sequence of the light chain variable region is set forth in SEQ ID NO: 8.

The amino acid sequences of the 3 CDR regions of the heavy chain variable region are identical to those of 4G10.

The amino acid sequences of the 3 CDR regions of the light chain variable region are identical to those of 4G10.

### (5) 4G10H3L3

An amino acid sequence of the heavy chain variable region is set forth in SEQ ID NO: 10, and an amino acid sequence of the light chain variable region is set forth in SEQ ID NO: 12. The amino acid sequences of the 3 CDR regions of the heavy chain variable region are identical to those of 4G10.

The amino acid sequences of the 3 CDR regions of the light chain variable region are identical to those of 4G10.

### (6) BiAb001(M)

Amino acid sequences of the 9 CDR regions associated with the heavy chain variable region are as follows:
HCDR1: GFAFSSYD (SEQ ID NO: 27)
HCDR2: ISGGGRYT (SEQ ID NO: 28)
HCDR3: ANRYGEAWFAY (SEQ ID NO: 29)
HCDR4: GYSFTGYT (SEQ ID NO: 33)
HCDR5: INPYNNIT (SEQ ID NO: 34)
HCDR6: ARLDYRSY (SEQ ID NO: 35)
HCDR7: TGAVTTSNF (SEQ ID NO: 36)
HCDR8: GTN (SEQ ID NO: 37)
HCDR9: ALWYSNHWV (SEQ ID NO: 38)

Amino acid sequences of the 3 CDR regions associated with the light chain variable region are as follows:
LCDR1: QDINTY (SEQ ID NO: 30)
LCDR2: RAN (SEQ ID NO: 31)
LCDR3: LQYDEFPLT (SEQ ID NO: 32)

### (7) BiAb002(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

### (8) BiAb003(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

### (9) BiAb004(M)

The amino acid sequences of the 9 CDR regions associated with the heavy chain variable region are identical to those of BiAb001(M).

The amino acid sequences of the 3 CDR regions associated with the light chain variable region are identical to those of BiAb001(M).

For the antibody BiAb004(hG1TM) of the present invention, amino acid mutations are introduced into the non-variable region of BiAb004(M). According to the EU numbering system, amino acid mutations are introduced at positions 234, 235 and 237:
BiAb004(hG 1 TM) is obtained by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of CTLA4 protein (cytotoxic T-lymphocyte-associated antigen 4), it includes but is not limited to the full length of CTLA4 protein, or the extracellular fragment CTLA4 ECD of CTLA4 or a fragment comprising CTLA4 ECD; also included are fusion proteins of CTLA4 ECD, such as a fragment fused to an Fc protein fragment of a mouse or human IgG (mFc or hFc). However, those skilled in the art will appreciate that in the amino acid sequence of the CTLA4 protein, mutations or variations (including but not limited to, substitutions, deletions, and/or additions) can be naturally produced or artificially introduced without affecting biological functions thereof. Therefore, in the present invention, the term "CTLA4 protein" shall include all such sequences, including their natural or artificial variants. In addition, when describing a sequence fragment of the CTLA4 protein, it also includes the corresponding sequence fragments in their natural or artificial variants.

As used herein, when referring to the amino acid sequence of PD-1 protein, it includes but is not limited to the full length of PD-1 protein (NCBI GenBank: NM_005018), or the extracellular fragment PD-1 ECD of PD-1 or a fragment comprising PD-1 ECD; also included are fusion proteins of PD-1 ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions, and/or additions) can be naturally produced or artificially introduced without affecting biological functions thereof. Therefore, in the present invention, the term "PD-1 protein" should include all such sequences, including their natural or artificial variants. In addition, when describing a sequence fragment of the PD-1 protein, it also includes the corresponding sequence fragments in their natural or artificial variants.

As used herein, unless otherwise specified, the B7 is B7-1 and/or B7-2; specific protein sequences thereof are those known in the prior art, and reference may be made to sequences disclosed in the existing literature or GenBank. For example, B7-1 (CD80, NCBI Gene ID: 941); B7-2 (CD86, NCBI Gene ID: 942).

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, δ, γ, α and ε, and the antibodies are defined as IgM, IgD, IgG, IgA and IgE isotypes, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (called complementarity determining regions, or CDRs), and conservative regions called framework regions (FRs) are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form an antibody binding site. The assignment of amino acids to each region or domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. In particular, the heavy chain may further comprise more than 3 CDRs, such as 6, 9, or 12. For example, in the bifunctional antibody of the present invention, the heavy chain may be an scFv with the C terminus of the heavy chain of IgG antibody linked to another antibody, and in this case, the heavy chain comprises 9 CDRs. The term "antibody" is not limited by any specific method for producing the antibody. For example, the antibody includes, in particular, a recombinant antibody, a monoclonal antibody and a polyclonal antibody. The antibody may be antibodies of different isotypes, such as IgG (e.g., subtype IgG1, IgG2, IgG3, or IgG4), IgA1, IgA2, IgD, IgE, or IgM. Antigen-binding fragments (e.g., the above mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies.

As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technique first reported by Kohler et al. (Kohler et al., Nature, 256:495,1975), but can also be obtained using DNA recombination (see, e.g., U.S. Patent No. 4,816,567).

As used herein, the term "humanized antibody" refers to an antibody or antibody fragment obtained when all or a part of CDR regions of a human immunoglobulin (receptor antibody) is replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21: 397-402 (2000).

As used herein, the term "epitope" refers to a site on the antigen that an immunoglobulin or antibody specifically binds to. "Epitope" is also referred to in the art as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, an epitope typically comprises at least 3, 4, 5, 6, 7, 8, 9, 10, 11, 12, 13, 14 or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "E. coli expression system" refers to an expression system consisting of E. coli (strain) and a vector, wherein the E. coli (strain) is derived from a commercially available strain, such as but not limited to GI698, ER2566, BL21 (DE3), B834 (DE3), and BLR (DE3).

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *Escherichia coli* or *bacillus subtilis,* fungal cells such as yeast cells or *Aspergillus,* insect cells such as S2 drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) less than about 10⁻⁵ M, such as less than about 10⁻⁶ M, 10⁻¹ M, 10⁻⁸ M, 10⁻⁹ M or 10⁻¹⁰ M or less. In some embodiments of the present invention, the term "target" refers to specific binding.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less, for example, as measured by a BIACORE surface plasmon resonance (SPR) instrument or a Fortebio Octet molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount (e.g., for a disease associated with binding of CTLA4 to B7 or CTLA4 overactivity, such as a tumor) is an amount sufficient to prevent, stop, or delay the onset of the disease (e.g., a disease associated with binding of CTLA4 to B7 or CTLA4 overactivity, such as a tumor); a therapeutically effective amount is an amount sufficient to cure or at least partially stop a disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

As used herein, the term "completely eliminated" refers to the absence of binding signal or an extremely weak binding signal as detected by an existing instrument (e.g., a Fortebio Octet molecular interaction instrument). In one embodiment of the present invention, the absence of binding signal or the extremely weak binding signal refers to a binding signal (i.e., response value or Response) below 0.1 nm.

In the present invention, the terms "first" (e.g., first protein functional region, first linker fragment or first product) and "second" (e.g., second protein functional region, second linker fragment or second product) are used for distinguishing or clarity in expression and do not carry typical sequential meanings, unless otherwise specified.

In the present invention, "about" or "approximately" refers to that the value or physical quantity defined fluctuates within a range of 10%, 20% or 30%, unless otherwise specified. For example, about 100 minutes or approximately 100 minutes may be 90 minutes to 110 minutes, 80 minutes to 120 minutes or 70 minutes to 130 minutes.

Crystalline forms of chiauranib include, but are not limited to, non-solvated crystalline form A, non-solvated crystalline form B, and non-solvated crystalline form C. Description in Chinese granted patent CN 107868044 B is referred to, wherein:
the X-ray powder diffraction pattern of the non-solvated crystalline form A has characteristic peaks at reflection angles 20 of 6.88°, 9.26°, 12.74°, 13.82°, 18.58°, 20.86° and 25.72°;
the X-ray powder diffraction pattern of the non-solvated crystalline form B has characteristic peaks at reflection angles 20 of 4.88°, 9.68°, 12.74°, 14.52°, 17.72°, 24.30° and 25.26°;
the X-ray powder diffraction pattern of the non-solvated crystalline form C has characteristic peaks at reflection angles 20 of 4.84°, 9.68°, 12.92°, 14.60°, 16.46°, 17.44°, 22.00° and 25.28°.

### Beneficial effects of the present invention

The present invention achieves one or more of the following technical effects (1) to (2):
(1) The therapeutic combination of the present invention has a good effect on treating or preventing a tumor.
(2) In the therapeutic combination of the present invention, the anti-CTLA4-anti-PD-1 bispecific antibody and chiauranib have a synergistic effect, so that a synergistic effect on treating or preventing a tumor is achieved, wherein the tumor includes but is not limited to: melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, or nasopharyngeal cancer; preferably, the lung cancer is non-small cell lung cancer, small cell lung cancer or squamous cell lung cancer; preferably, the gastric cancer is gastric adenocarcinoma or gastroesophageal junction adenocarcinoma.

### BRIEF DESCRIPTION OF THE DRAWINGS

FIG. 1: Affinity constant assay of BiAb004(hG 1 TM) to FcyRI. The antibody concentrations for the curve pairs from top to bottom are 50 nM, 25 nM, 12.5 nM, 6.25 nM and 3.12 nM, respectively.
FIG. 2: Affinity constant assay of BiAb004(hG 1 WT) to FcyRI. The antibody concentrations for the curve pairs from top to bottom are 50 nM, 25 nM, 12.5 nM, 6.25 nM and 3.12 nM, respectively.
FIG. 3: Affinity constant assay of BiAb004(hG 1 TM) to FcγRIIIa_V158. The antibody concentrations for the curve pairs from top to bottom are 500 nM, 250 nM, 125 nM, 62.5 nM and 31.25 nM, respectively.
FIG. 4: Affinity constant assay of BiAb004(hG 1 WT) to FcγRIIIa_V158. The antibody concentrations for the curve pairs from top to bottom are 500 nM, 250 nM, 125 nM, 62.5 nM and 31.25 nM, respectively.
FIG. 5: Affinity constant assay of BiAb004(hG 1 TM) to FcγRIIIa_F158. The antibody concentrations for the curve pairs from top to bottom are 500 nM, 250 nM, 125 nM, 62.5 nM and 31.25 nM, respectively.
FIG. 6: Affinity constant assay of BiAb004(hG1WT) to FcγRIIIa_F158. The antibody concentrations for the curve pairs from top to bottom are 500 nM, 250 nM, 125 nM, 62.5 nM and 31.25 nM, respectively.
FIG. 7: Affinity constant assay of BiAb004(hG1TM) to FcγRIIa_H131. The antibody concentrations for the curve pairs from top to bottom are 200 nM, 100 nM, 50 nM, 25 nM and 12.5 nM, respectively.
FIG. 8: Affinity constant assay of BiAb004(hG 1 WT) to FcγRIIa_H131. The antibody concentrations for the curve pairs from top to bottom are 200 nM, 100 nM, 50 nM, 25 nM and 12.5 nM, respectively.
FIG. 9: Affinity constant assay of BiAb004(hG1TM) to FcyRIIa_R131. The antibody concentrations for the curve pairs from top to bottom are 200 nM, 100 nM, 50 nM, 25 nM and 12.5 nM, respectively.
FIG. 10: Affinity constant assay of BiAb004(hG1 WT) to FcyRIIa_R131. The antibody concentrations for the curve pairs from top to bottom are 200 nM, 100 nM, 50 nM, 25 nM and 12.5 nM, respectively.
FIG. 11: Affinity constant assay of BiAb004(hG1TM) to FcyRIIb. The antibody concentrations for the curve pairs from top to bottom are 200 nM, 100 nM, 50 nM, 25 nM and 12.5 nM, respectively.
FIG. 12: Affinity constant assay of BiAb004(hG1WT) to FcyRIIb. The antibody concentrations for the curve pairs from top to bottom are 200 nM, 100 nM, 50 nM, 25 nM and 12.5 nM, respectively.
FIG. 13: Affinity constant assay of BiAb004(hG1TM) to C1q. The antibody concentrations for the curve pairs from top to bottom are 20 nM, 10 nM, 5 nM, 2.5 nM and 1.25 nM, respectively.
FIG. 14: Affinity constant assay of BiAb004(hG1WT) to C1q. The antigen concentrations for the curve pairs from top to bottom are 20 nM, 10 nM, 5 nM, 2.5 nM and 1.25 nM, respectively.
FIG. 15: Affinity constant assay of 5C10H2L2-IgG1mt to C1q. The antigen concentrations for the curve pairs from top to bottom are 20 nM, 10 nM, 5 nM, 2.5 nM and 1.25 nM, respectively.
FIG. 16: ADCC activity assay of BiAb004(hG1WT) and BiAb004(hG1TM) on 293T-CTLA4-PD1 cells expressing CTLA-4 and PD-1 antigens.
FIG. 17: IFN-y secretion by mixed lymphocytes induced by BiAb004(hG1TM) in combination with chiauranib.
FIG. 18: IL-2 secretion by mixed lymphocytes induced by BiAb004(hG1TM) in combination with chiauranib.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

In the following examples of the present invention:
BALB/c mice were purchased from Guangdong Medical Laboratory Animal Center.
Human peripheral blood mononuclear cells were isolated and prepared in Akeso Biopharma Inc., with informed consent of the donor.
Raji-PDL1 is a cell expressing human PD-L1 constructed by Akeso Biopharma Inc. on the basis of human B cell line cell Raji via transfection.
Ficoll-Paque^{™} PLUS (or Ficoll-Paque PLUS) was purchased from GE Healthcare.
Human IL-2 ELISA kit was purchased from Dakewe Biotech Co., Ltd.
RPMI 1640 medium, DMEM medium, Trypsin-EDTA (0.25%) phenol red and Blasticidin were all purchased from Gibco.
FBS was purchased from Excell bio.

The sequence of the isotype control antibody, human anti-hen egg lysozyme IgG (anti-HEL antibody, or human IgG, abbreviated as hIgG) is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-146).

In the following examples of the present invention, the chiauranib used was derived from Shenzhen Chipscreen Biosciences Co., Ltd.

### Preparation Example 1: Sequence Design of Anti-CTLA4 Antibodies

The amino acid sequences and encoding nucleotide sequences of the heavy and light chains of the anti-CTLA4 antibody 4G10 and its humanized antibodies 4G10H1L1 and 4G10H3L3 are identical to those of 4G10, 4G10H1L1 and 4G10H3L3 in Chinese Patent Publication No. CN106967172A, respectively.
(1) Heavy and light chain variable region sequences of 4G10
   Nucleotide sequence of the heavy chain variable region: (372 bp) 
CAGGTCAAGCTGCAGGAGTCTGGACCTGAGCTGGTGAAGCCTGGAGCTTCAAT
GAAGATATCCTGCAAGGCTTCTGGTTACTCATTCACTGGCTACACCATGAACTG
GGTGAAGCAGAGCCATGGAAAGAACCTTGAATGGATTGGACTTATTAATCCTTA
CAATAATATTACTAACTACAACCAGAAGTTCATGGGCAAGGCCACATTTACTGTA
GACAAGTCATCCAGCACAGCCTACATGGAACTCCTCAGACTGACATCTGAAGAC
TCTGGAGTCTATTTCTGTGCAAGACTCGACTATAGGTCTTATTGGGGCCAAGGG
ACTCTGGTCACTGTCTCTGCAGCCAAAACGACACCCCCATCTGTCTAT (SEQ ID
NO: 1)
   The encoded amino acid sequence: (124 aa) 
QVKLQESGPELVKPGASMKISCKASGYSFTGYTMNWVKQSHGKNLEWIGLINPYN
NITNYNQKFMGKATFTVDKSSSTAYMELLRLTSEDSGVYFCARLDYRSYWGQGTLV
TVSAAKTTPPSVY (SEQ ID NO: 2)
   Nucleotide sequence of the light chain variable region: (378 bp) 
CAGGCTGTTGTGACTCAGGAATCTGCACTCACCACATCACCTGGTGAAACAGTC
ACACTCACTTGTCGCTCAAGTACTGGGGCTGTTACAACTAGTAACTTTGCCAAC
TGGGTCCAAGAAAAACCAGATCATTTATTCACTAGTCTAATAGGTGGTACCAACA
ACCGAGCTCCAGGTGTTCCTGCCAGATTCTCAGGCTCCCTGATTGGAGACAAG
GCTGCCCTCACCATCACAGGGGCACAGACTGAGGATGAGGCAATATATTTCTGT
GCTCTATGGTACAGCAACCATTGGGTGTTCGGTGGAGGAACCAAACTGACTGTC
CTAGGCCAGCCCAAGTCTTCGCCATCAGTCACCCTGTTTCAAGGGCAATTCTGC
(SEQ ID NO: 3)
   The encoded amino acid sequence: (126 aa) 
QAVVTQESALTTSPGETVTLTCRSSTGAVTTSNFANWVQEKPDHLFTSLIGGTNNRA
PGVPARFSGSLIGDKAALTITGAQTEDEAIYFCALWYSNHWVFGGGTKLTVLGQPK
 SSPSVTLFQGQFC(SEQ ID NO: 4)
(2) Heavy and light chain variable region sequences of humanized monoclonal antibody 4G10H1L1
   Nucleotide sequence of the heavy chain variable region (4G10H1V): (345 bp) 
CAGGTGCAGCTGGTGGAGTCTGGGGCCGAGCTGGTGAAGCCCGGCGCCTCCAT
GAAGATCTCTTGCAAGGCCAGCGGATACAGTTTCACTGGCTATACCATGAACTG
GGTCAAACAGGCTCCAGGACAGGGACTGGAGTGGATCGGGCTGATTAATCCTT
ACAACAACATCACCAACTACAACCAGAAGTTCATGGGAAAAGCAACCTTTACAG
TGGACAAGAGCATTTCCACAGCCTACATGGAACTGAGCCGGCTGACTTCAGAC
GATAGCGGGGTCTATTTTTGTGCAAGGCTGGATTATCGCTCTTACTGGGGGCAG
GGAACTCTGGTCACTGTCTCCGCT(SEQ ID NO: 5)
   The encoded amino acid sequence: (115 aa) 
QVQLVESGAELVKPGASMKISCKASGYSFTGYTMNWVKQAPGQGLEWIGLINPYN
NITNYNQKFMGKATFTVDKSISTAYMELSRLTSDDSGVYFCARLDYRSYWGQGTLV
TVSA(SEQ ID NO: 6)
   Nucleotide sequence of the light chain variable region (4G10L1V): (327 bp) 
CAGGCTGTCGTCACTCAGGAACCTTCACTGACTGTGAGCCCAGGAGGAACTGT
CACCCTGACATGCGGAAGCTCCACCGGAGCAGTGACCACATCCAACTTCGCCA
ATTGGGTCCAGGAAAAGCCAGGCCAGGCATTTCGATCCCTGATCGGAGGCACA
AACAATCGGGCTTCTTGGGTGCCCGCAAGATTCTCAGGAAGCCTGCTGGGGGG
AAAAGCCGCTCTGACCATTAGTGGCGCTCAGCCTGAGGACGAAGCCGAGTACT
TCTGCGCTCTGTGGTATAGCAACCACTGGGTGTTTGGCGGGGGAACAAAGCTG
ACTGTGCTG(SEQ ID NO: 7)
   The encoded amino acid sequence: (109 aa) 
QAVVTQEPSLTVSPGGTVTLTCGSSTGAVTTSNFANWVQEKPGQAFRSLIGGTNNRA
SWVPARFSGSLLGGKAALTISGAQPEDEAEYFCALWYSNHWVFGGGTKLTVL(SEQ
ID NO: 8)
(3) Heavy and light chain variable region sequences of humanized monoclonal antibody 4G10H3L3
   Nucleotide sequence of the heavy chain variable region (4G10H3V): (345 bp) 
CAGGTGCAGCTGGTCGAGTCTGGGGCCGAAGTGAAGAAACCCGGCGCCTCAGT
GAAGGTCAGCTGCAAGGCCAGCGGGTACAGTTTCACTGGATATACCATGAACTG
GGTCCGACAGGCCCCTGGCCAGGGGCTGGAGTGGATCGGCCTGATTAACCCTT
ACAACAACATCACTAACTACGCACAGAAGTTCCAGGGGAGAGTGACCTTTACAG
TGGACACCAGCATTTCCACAGCCTACATGGAACTGTCCCGGCTGAGATCTGACG
ATACAGGCGTGTACTTCTGCGCTAGGCTGGATTACCGCAGCTATTGGGGACAGG
GCACACTGGTGACTGTCAGCGCA(SEQ ID NO: 9)
   The encoded amino acid sequence (4G10H3V): (115 aa) 
QVQLVESGAEVKKPGASVKVSCKASGYSFTGYTMNWVRQAPGQGLEWIGLINPYN
NITNYAQKFQGRVTFTVDTSISTAYMELSRLRSDDTGVYFCARLDYRSYWGQGTLV
TVSA(SEQ ID NO: 10)
   Nucleotide sequence of the light chain variable region (4G10L3V): (327 bp) 
CAGGCTGTCGTCACTCAGGAACCTTCACTGACCGTGTCTCCTGGCGGGACTGT
CACCCTGACATGCGGCAGCTCCACAGGGGCCGTGACCACAAGTAACTTCCCAA
ATTGGGTCCAGCAGAAGCCAGGACAGGCTCCCCGGAGTCTGATCGGAGGCACC
AACAACAAGGCCAGCTGGACACCCGCACGGTTCAGCGGCAGCCTGCTGGGCG
GCAAGGCCGCTCTGACAATTAGCGGAGCCCAGCCTGAGGACGAAGCCGAGTAC
TATTGCGCTCTGTGGTACTCCAACCACTGGGTGTTCGGCGGCGGCACCAAGCT
GACTGTGCTG(SEQ ID NO: 11)
   The encoded amino acid sequence (4G10L3V): (109 aa)

QAVVTQEPSLTVSPGGTVTLTCGSSTGAVTTSNFPNWVQQKPGQAPRSLIGGTNNK
ASWTPARFSGSLLGGKAALTISGAQPEDEAEYYCALWYSNHWVFGGGTKLTVL(SE
Q ID NO: 12)

### Preparation Example 2: Sequence Design of Anti-PD-1 Antibody 14C12 and Its Humanized Antibody 14C12H1L1

The amino acid sequences and encoding nucleotide sequences of the heavy and light chains of the anti-PD-1 antibody 14C12 and its humanized antibodies 14C12H1L1 are identical to those of 14C12 and 14C12H1L1 in Chinese Patent Publication No. CN 106967172A, respectively.
(1) Heavy and light chain variable region sequences of 14C12
   Nucleotide sequence of the heavy chain variable region: (354 bp) 
GAGGTCAAACTGGTGGAGAGCGGCGGCGGGCTGGTGAAGCCCGGCGGGTCAC
TGAAACTGAGCTGCGCCGCTTCCGGCTTCGCCTTTAGCTCCTACGACATGTCAT
GGGTGAGGCAGACCCCTGAGAAGCGCCTGGAATGGGTCGCTACTATCAGCGGA
GGCGGGCGATACACCTACTATCCTGACTCTGTCAAAGGGAGATTCACAATTAGT
CGGGATAACGCCAGAAATACTCTGTATCTGCAGATGTCTAGTCTGCGGTCCGAG
GATACAGCTCTGTACTATTGTGCAAACCGGTACGGCGAAGCATGGTTTGCCTATT
GGGGACAGGGCACCCTGGTGACAGTCTCTGCC(SEQ ID NO: 13)
   The encoded amino acid sequence: (118 aa) 
EVKLVESGGGLVKPGGSLKLSCAASGFAFSSYDMSWVRQTPEKRLEWVATISGGGR
YTYYPDSVKGRFTISRDNARNTLYLQMSSLRSEDTALYYCANRYGEAWFAYWGQGT
LVTVSA(SEQ ID NO: 14)
   Nucleotide sequence of the light chain variable region: (321 bp) 
GACATTAAGATGACACAGTCCCCTTCCTCAATGTACGCTAGCCTGGGCGAGCGA
GTGACCTTCACATGCAAAGCATCCCAGGACATCAACACATACCTGTCTTGGTTT
CAGCAGAAGCCAGGCAAAAGCCCCAAGACCCTGATCTACCGGGCCAATAGACT
GGTGGACGGGGTCCCCAGCAGATTCTCCGGATCTGGCAGTGGGCAGGATTACT
CCCTGACCATCAGCTCCCTGGAGTATGAAGACATGGGCATCTACTATTGCCTGC
AGTATGATGAGTTCCCTCTGACCTTTGGAGCAGGCACAAAACTGGAACTGAAG(
SEQ ID NO: 15)
   The encoded amino acid sequence: (107 aa) 
DIKMTQSPSSMYASLGERVTFTCKASQDINTYLSWFQQKPGKSPKTLIYRANRLVD
GVPSRFSGSGSGQDYSLTISSLEYEDMGIYYCLQYDEFPLTFGAGTKLELK(SEQ ID
NO: 16)
(2) Heavy and light chain variable region sequences and heavy and light chain sequences of humanized monoclonal antibody 14C12H1L1
   Nucleotide sequence of the heavy chain variable region: (354 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTCT
AGAGATAACAGTAAGAACAATCTGTATCTGCAGATGAACAGCCTGAGGGCTGAG
GACACCGCACTGTACTATTGTGCCAACCGCTACGGGGAAGCATGGTTTGCCTAT
TGGGGGCAGGGAACCCTGGTGACAGTCTCTAGT (SEQ ID NO: 17)
   The encoded amino acid sequence: (118 aa) 
EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWGQ
GTLVTVSS(SEQ ID NO: 18)
   Nucleotide sequence of the light chain variable region: (321 bp) 
GACATTCAGATGACTCAGAGCCCCTCCTCCATGTCCGCCTCTGTGGGCGACAGG
GTCACCTTCACATGCCGCGCTAGTCAGGATATCAACACCTACCTGAGCTGGTTT
CAGCAGAAGCCAGGGAAAAGCCCCAAGACACTGATCTACCGGGCTAATAGACT
GGTGTCTGGAGTCCCAAGTCGGTTCAGTGGCTCAGGGAGCGGACAGGACTACA
CTCTGACCATCAGCTCCCTGCAGCCTGAGGACATGGCAACCTACTATTGCCTGC
AGTATGATGAGTTCCCACTGACCTTTGGCGCCGGGACAAAACTGGAGCTGAAG(
SEQ ID NO: 19)
   The encoded amino acid sequence: (107 aa) 
DIQMTQSPSSMSASVGDRVTFTCRASQDINTYLSWFQQKPGKSPKTLIYRANRLVSG
VPSRFSGSGSGQDYTLTISSLQPEDMATYYCLQYDEFPLTFGAGTKLELK(SEQ ID
NO: 20)
   DNA sequence of 14C12H1L1 heavy chain (14C12H1): (1344 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTCT
AGAGATAACAGTAAGAACAATCTGTATCTGCAGATGAACAGCCTGAGGGCTGAG
GACACCGCACTGTACTATTGTGCCAACCGCTACGGGGAAGCATGGTTTGCCTAT
TGGGGGCAGGGAACCCTGGTGACAGTCTCTAGTGCCAGCACCAAAGGACCTAG
CGTGTTTCCTCTCGCCCCCTCCTCCAAAAGCACCAGCGGAGGAACCGCTGCTC
TCGGATGTCTGGTGAAGGACTACTTCCCTGAACCCGTCACCGTGAGCTGGAATA
GCGGCGCTCTGACAAGCGGAGTCCATACATTCCCTGCTGTGCTGCAAAGCAGC
GGACTCTATTCCCTGTCCAGCGTCGTCACAGTGCCCAGCAGCAGCCTGGGCAC
CCAGACCTACATCTGTAACGTCAACCACAAGCCCTCCAACACCAAGGTGGACAA
GAAAGTGGAGCCCAAATCCTGCGACAAGACACACACCTGTCCCCCCTGTCCTG
CTCCCGAACTCCTCGGAGGCCCTAGCGTCTTCCTCTTTCCTCCCAAACCCAAGG
ACACCCTCATGATCAGCAGAACCCCTGAAGTCACCTGTGTCGTCGTGGATGTCA
GCCATGAGGACCCCGAGGTGAAATTCAACTGGTATGTCGATGGCGTCGAGGTG
CACAACGCCAAAACCAAGCCCAGGGAGGAACAGTACAACTCCACCTACAGGGT
GGTGTCCGTGCTGACAGTCCTCCACCAGGACTGGCTGAACGGCAAGGAGTACA
AGTGCAAGGTGTCCAACAAGGCTCTCCCTGCCCCCATTGAGAAGACCATCAGC
AAGGCCAAAGGCCAACCCAGGGAGCCCCAGGTCTATACACTGCCTCCCTCCAG
GGACGAACTCACCAAGAACCAGGTGTCCCTGACCTGCCTGGTCAAGGGCTTTT
ATCCCAGCGACATCGCCGTCGAGTGGGAGTCCAACGGACAGCCCGAGAATAAC
TACAAGACCACCCCTCCTGTCCTCGACTCCGACGGCTCCTTCTTCCTGTACAGC
AAGCTGACCGTGGACAAAAGCAGGTGGCAGCAGGGAAACGTGTTCTCCTGCAG
CGTGATGCACGAAGCCCTCCACAACCACTACACCCAGAAAAGCCTGTCCCTGA
GCCCCGGCAAA(SEQ ID NO: 21)
   The encoded amino acid sequence: (448 aa) 
EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWGQ
GTLVTVSSASTKGPSVFPLAPSSKSTSGGTAALGCLVKDYFPEPVTVSWNSGALTSG
 
VHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 22)
   DNA sequence of 14C12H1L1 light chain (14C12L1): (642 bp) 
GACATTCAGATGACTCAGAGCCCCTCCTCCATGTCCGCCTCTGTGGGCGACAGG
GTCACCTTCACATGCCGCGCTAGTCAGGATATCAACACCTACCTGAGCTGGTTT
CAGCAGAAGCCAGGGAAAAGCCCCAAGACACTGATCTACCGGGCTAATAGACT
GGTGTCTGGAGTCCCAAGTCGGTTCAGTGGCTCAGGGAGCGGACAGGACTACA
CTCTGACCATCAGCTCCCTGCAGCCTGAGGACATGGCAACCTACTATTGCCTGC
AGTATGATGAGTTCCCACTGACCTTTGGCGCCGGGACAAAACTGGAGCTGAAG
CGAACTGTGGCCGCTCCCTCCGTCTTCATTTTTCCCCCTTCTGACGAACAGCTG
AAATCAGGCACAGCCAGCGTGGTCTGTCTGCTGAACAATTTCTACCCTAGAGAG
GCAAAAGTGCAGTGGAAGGTCGATAACGCCCTGCAGTCCGGCAACAGCCAGGA
GAGTGTGACTGAACAGGACTCAAAAGATAGCACCTATTCCCTGTCTAGTACACT
GACTCTGTCCAAGGCTGATTACGAGAAGCACAAAGTGTATGCATGCGAAGTGAC
ACATCAGGGACTGTCAAGCCCCGTGACTAAGTCTTTTAACCGGGGCGAATGT(SE
Q ID NO: 23)
   The encoded amino acid sequence: (214 aa)

DIQMTQSPSSMSASVGDRVTFTCRASQDINTYLSWFQQKPGKSPKTLIYRANRLVSG
VPSRFSGSGSGQDYTLTISSLQPEDMATYYCLQYDEFPLTFGAGTKLELKRTVAAPS
VFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC(SEQ ID NO: 24)

### Preparation Example 3: Sequence Design of Bifunctional Antibodies BiAb001(M), BiAb002(M). BiAb003(M) and BiAb004(M)

The structural pattern of the bifunctional antibodies BiAb001(M), BiAb002(M), BiAb003(M) and BiAb004(M) is in the Morrison format (IgG-scFv), i.e., C termini of two heavy chains of one IgG antibody are linked to the scFv fragment of another antibody via linker fragments. The components for the heavy and light chain design are shown in Table A below.

**Table A: Sequence design of BiAb001(M), BiAb002(M), BiAb003(M) and BiAb004(M)**

| Bifunctional antibody | Immunoglobulin portion | | Linker fragment | scFv moiety |
|---|---|---|---|---|
| | Heavy chain | Light chain | | |
| BiAb001(M) | 14C12H1 | 14C12L1 | Linker1 | 4G10H1V(M)- Linker2 - 4G10L1V(M) |
| BiAb002(M) | 14C12H1 | 14C12L1 | Linker2 | 4G10H1V(M)-Linker2 -4G10L1V(M) |
| BiAb003(M) | 14C12H1 | 14C12L1 | Linker1 | 4G10H3V(M)- Linker2 - 4G10L3V(M) |
| BiAb004(M) | 14C12H1 | 14C12L1 | Linker2 | 4G10H3V(M)- Linker2 - 4G10L3V(M) |

In Table A above:
The amino acid sequence of Linker1 is (GGGGS)3 (SEQ ID NO: 25).
The amino acid sequence of Linker2 is (GGGGS)4 (SEQ ID NO: 26).

In Table A above, scFv fragments 4G10H1V(M), 4G10L1V(M), 4G10H3V(M) and 4G10L3V(M) of BiAb001(M), BiAb002(M), BiAb003(M) and BiAb004(M) antibodies comprise mutations in individual amino acids of framework regions on the basis of 4G10H1V, 4G10L1V, 4G10H3V and 4G10L3V, respectively, which effectively optimizes the structure of the antibodies and improves their efficacy.
(1) 4G10H1V(M): (115 aa, mutation sites underlined in the amino acid sequence based on 4G10H1V) 
QVQLVESGAELVKPGASMKISCKASGYSFTGYTMNWVKQAPGQCLEWIGLINPYN
NITNYNQKFMGKATFTVDKSISTAYMELSRLTSDDSGVYFCARLDYRSYWGQGTLV
TVSA(SEQ ID NO: 41)
(2) 4G10L1V(M): (110 aa, mutation sites underlined in the amino acid sequence based on 4G10L1V) 
QAVVTQEPSLTVSPGGTVTLTCGSSTGAVTTSNFANWVQEKPGQAFRSLIGGTNNRA
SWVPARFSGSLLGGKAALTISGAQPEDEAEYFCALWYSNHWVFGCGTKLTVLR(SE
Q ID NO: 42)
(3) 4G10H3V(M): (115 aa, mutation sites underlined in the amino acid sequence based on 4G10H3V) 
QVQLVESGAEVKKPGASVKVSCKASGYSFTGYTMNWVRQAPGQCLEWIGLINPYN
NITNYAQKFQGRVTFTVDTSISTAYMELSRLRSDDTGVYFCARLDYRSYWGQGTLV
TVSA(SEQ ID NO: 43)
(4) 4G10L3V(M): (110 aa, mutation sites underlined in the amino acid sequence based on 4G10L3V)

QAVVTQEPSLTVSPGGTVTLTCGSSTGAVTTSNFPNWVQQKPGQAPRSLIGGTNNK
ASWTPARFSGSLLGGKAALTISGAQPEDEAEYYCALWYSNHWVFGCGTKLTVLR(S
EQ ID NO: 44)

For distinguishment from the mutated antibodies hereinafter, BiAb004(M) is also referred to as BiAb004(hG1WT) in the examples of the present invention. BiAb004(M) described above is the "wild-type", comprising an Ig gamma-1 chain C region (ACCESSION: P01857) as the heavy chain constant region and an Ig kappa chain C region (ACCESSION: P01834) as the light chain constant region.

### Preparation Example 4: Non-Variable Region Amino Acid Mutation Design Based on Humanized Bifunctional Antibody BiAb004

On the basis of BiAb004(hG1WT) obtained in Preparation Example 3, BiAb004(hG 1 TM) was obtained by introducing a leucine-to-alanine point mutation at position 234 (L234A), a leucine-to-alanine point mutation at position 235 (L235A), and a glycine-to-alanine point mutation at position 237 (G237A) in the heavy chain by the inventors.
DNA sequence of heavy chain of the immunoglobulin portion in BiAb004(hG1TM): (1344 bp, mutation sites underlined) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
 
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTCT
AGAGATAACAGTAAGAACAATCTGTATCTGCAGATGAACAGCCTGAGGGCTGAG
GACACCGCACTGTACTATTGTGCCAACCGCTACGGGGAAGCATGGTTTGCCTAT
TGGGGGCAGGGAACCCTGGTGACAGTCTCTAGTGCCAGCACCAAAGGGCCCAG
CGTGTTTCCTCTCGCCCCCTCCTCCAAAAGCACCAGCGGAGGAACCGCTGCTC
TCGGATGTCTGGTGAAGGACTACTTCCCTGAACCCGTCACCGTGAGCTGGAATA
GCGGCGCTCTGACAAGCGGAGTCCATACATTCCCTGCTGTGCTGCAAAGCAGC
GGACTCTATTCCCTGTCCAGCGTCGTCACAGTGCCCAGCAGCAGCCTGGGCAC
CCAGACCTACATCTGTAACGTCAACCACAAGCCCTCCAACACCAAGGTGGACAA
GAAAGTGGAGCCCAAATCCTGCGACAAGACACACACCTGTCCCCCCTGTCCTG
CTCCCGAAGCTGCTGGAGCCCCTAGCGTCTTCCTCTTTCCTCCCAAACCCAAGG
ACACCCTCATGATCAGCAGAACCCCTGAAGTCACCTGTGTCGTCGTGGATGTCA
GCCATGAGGACCCCGAGGTGAAATTCAACTGGTATGTCGATGGCGTCGAGGTG
CACAACGCCAAAACCAAGCCCAGGGAGGAACAGTACAACTCCACCTACAGGGT
GGTGTCCGTGCTGACAGTCCTCCACCAGGACTGGCTGAACGGCAAGGAGTACA
AGTGCAAGGTGTCCAACAAGGCTCTCCCTGCCCCCATTGAGAAGACCATCAGC
AAGGCCAAAGGCCAACCCAGGGAGCCCCAGGTCTATACACTGCCTCCCTCCAG
GGACGAACTCACCAAGAACCAGGTGTCCCTGACCTGCCTGGTCAAGGGCTTTT
ATCCCAGCGACATCGCCGTCGAGTGGGAGTCCAACGGACAGCCCGAGAATAAC
TACAAGACCACCCCTCCTGTCCTCGACTCCGACGGCTCCTTCTTCCTGTACAGC
AAGCTGACCGTGGACAAAAGCAGGTGGCAGCAGGGAAACGTGTTCTCCTGCAG
CGTGATGCACGAAGCCCTCCACAACCACTACACCCAGAAAAGCCTGTCCCTGA
GCCCCGGCAAA(SEQ ID NO: 39)
Amino acid sequence of heavy chain of the immunoglobulin portion in BiAb004(hG1TM): (448 aa, mutation sites underlined)

EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWGQ
GTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKT
HTCPPCPAPEAAGAPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 40)

BiAb004(hG1TM) and BiAb004(hG1WT) share the same DNA sequence of light chain and the same encoded amino acid sequence. The specific sequence is shown in Preparation Example 3.

### Experimental Example 1: Affinity Assay of BiAb004lhG1WTl and BiAb004(hG1TM) to Receptor FcγRI

The Fc receptor FcyRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody.

The affinity constants of BiAb004(hG1WT) and BiAb004(hG1TM) to FcyRI were measured in this experiment using a Fortebio Octet molecular interaction instrument to evaluate the potential ADCC and ADCP activities of the antibodies.

The method for determining the affinity constants of the corresponding antibodies to FcyRI by the Fortebio Octet molecular interaction instrument is briefly described as follows: The sample dilution buffer was a solution of PBS, 0.02% Tween-20 and 0.1% BSA at pH 7.4. An FcyRI solution at a concentration of 1 µg/mL (purchased from Sinobio) was added to the HIS1K sensor to immobilize FcyRI on the sensor surface for 50 s. The association and dissociation constants of the antibodies to FcyRI were both determined in the buffer with the antibody concentrations being 3.12-50 nM (serial two-fold dilution). The sensor with immobilized antigen was equilibrated in the buffer for 60 s, and then the binding of the immobilized FcyRI on the sensor to the antibodies was determined for 120 s; the dissociation of FcyRI from the antibodies was determined for 120 s. The temperature was 30 °C and the frequency was 0.3 Hz. The data were analyzed by 1:1 model fitting to obtain the affinity constants of the antibodies to FcyRI.

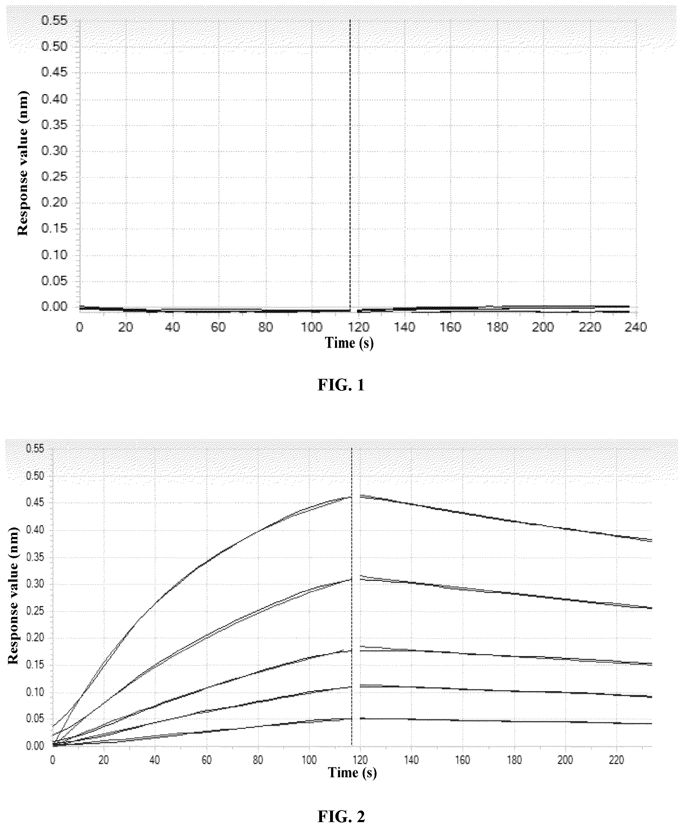

The results of affinity constant assay of BiAb004(hG1TM) and BiAb004(hG1WT) to FcyRI are shown in Table 1 and FIGs. 1 and 2 below.

**Table 1: Kinetics for binding of BiAb004(hG1WT) and BiAb004(hG1TM) to FcyRI**

| Sample ID | K_{D} (M) | kon (1/Ms) | S E (kon) | kdis (1/s) | S E(kdis) | Rmax (nm) |
|---|---|---|---|---|---|---|
| BiAb004 (hG1TM) | N/A | N/A | N/A | N/A | N/A | N/A |
| BiAb004 (hG1WT) | 5.92E-09 | 3.06E+05 | 8.35E+03 | 1.81E-03 | 5.75E-05 | 0.53-0.62 |

The results show that BiAb004(hG1WT) bound to FcyRI with an affinity constant of 5.92E-09 M; while for BiAb004(hG 1 TM), no corresponding data was obtained as no binding signal or an extremely weak signal to FcyRI was detected, indicating no binding to FcyRI.

The results suggest that the binding activity of BiAb004(hG1TM) to FcyRI is effectively eliminated as compared to BiAb004(hG 1 WT).

### Experimental Example 2: Affinity Constant Assay of BiAb004lhG1WTl and BiAb004lhG1TMl to FcγRIIIa V158

The Fc receptor FcγRIIIa_V158 (also known as CD16a_V158) can bind to the Fc fragment of an IgG antibody and mediate ADCC effects.

The affinity constants of BiAb004(hG1WT) and BiAb004(hG 1 TM) to FcγRIIIa_V158 were measured in the experiment using a Fortebio Octet molecular interaction instrument to evaluate the ADCC activity of the antibodies.

The method for determining the affinity constant of the corresponding antibodies to FcγRIIIa_V158 by the Fortebio Octet molecular interaction instrument is briefly described as follows: The sample dilution buffer was a solution of PBS, 0.02% Tween-20 and 0.1% BSA at pH 7.4. FcγRIIIa_V158 at 5 µg/mL was immobilized on the HIS1K sensor for 120 s. The sensor was equilibrated in a buffer for 60 s, and the binding of the immobilized FcγRIIIa_V158 on the sensor to the antibodies at concentrations of 31.25-500 nM (serial two-fold dilution) was determined for 60 s. The antibodies were dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.

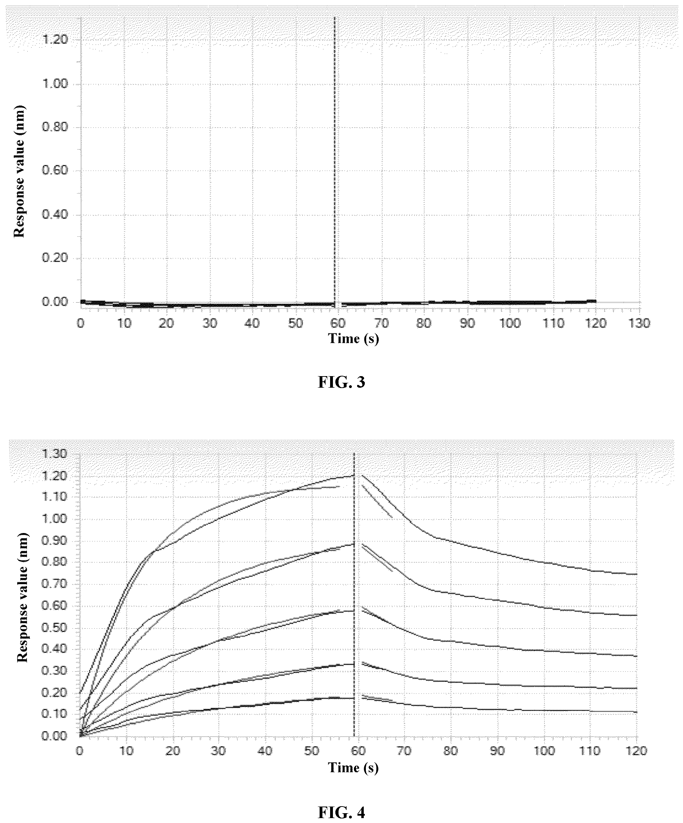

The results of affinity constant assay of BiAb004(hG1TM) and BiAb004(hG1WT) to FcγRIIIa_V158 are shown in Table 2 and FIGs. 3 and 4 below.

**Table 2: Kinetics for binding of BiAb004(hG1WT) and BiAb004(hG1TM) to FcyRIIIa V158**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 1.77E-07 | 1.21E+05 | 1.64E+04 | 2.14E-02 | 3.71E-03 | 1.56-1.61 |

The results show that BiAb004(hG1WT) bound to FcγRIIIa_V158 with an affinity constant of 1.77E-07 M; while for BiAb004(hG 1 TM), no corresponding data was obtained as no binding signal or an extremely weak signal to FcγRIIIa_V158 was detected, indicating no binding to FcγRIIIa_V158.

The results suggest that the binding activity of BiAb004(hG1TM) to FcyR IIIa_V158 is effectively eliminated as compared to BiAb004(hG 1 WT).

### Experimental Example 3: Affinity Constant Assay of BiAb004(hG1WT) and BiAb004(hG 1 TM) to FcγRIIIa F158

The Fc receptor FcγRIIIa_F158 (also known as CD16a_F158) can bind to the Fc fragment of an IgG antibody and mediate ADCC.

The affinity constants of BiAb004(hG 1 WT) and BiAb004(hG 1 TM) to FcγRIIIa_F158 were measured in this experiment using a Fortebio Octet molecular interaction instrument to evaluate the ADCC activity of the antibodies.

The method for determining the affinity constant of BiAb004(hG 1 WT) and BiAb004(hG 1 TM) to FcyRIIIa_F158 by the Fortebio Octet molecular interaction instrument is briefly described as follows: The sample dilution buffer was a solution of PBS, 0.02% Tween-20 and 0.1% BSA at pH 7.4. FcγRIIIa_F158 at 5 µg/mL was immobilized on the HIS1K sensor for 120 s. The sensor was equilibrated in a buffer for 60 s, and the binding of the immobilized FcγRIIIa_F158 on the sensor to the antibodies at concentrations of 31.25-500 nM (serial two-fold dilution) was determined for 60 s. The antibodies were dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.

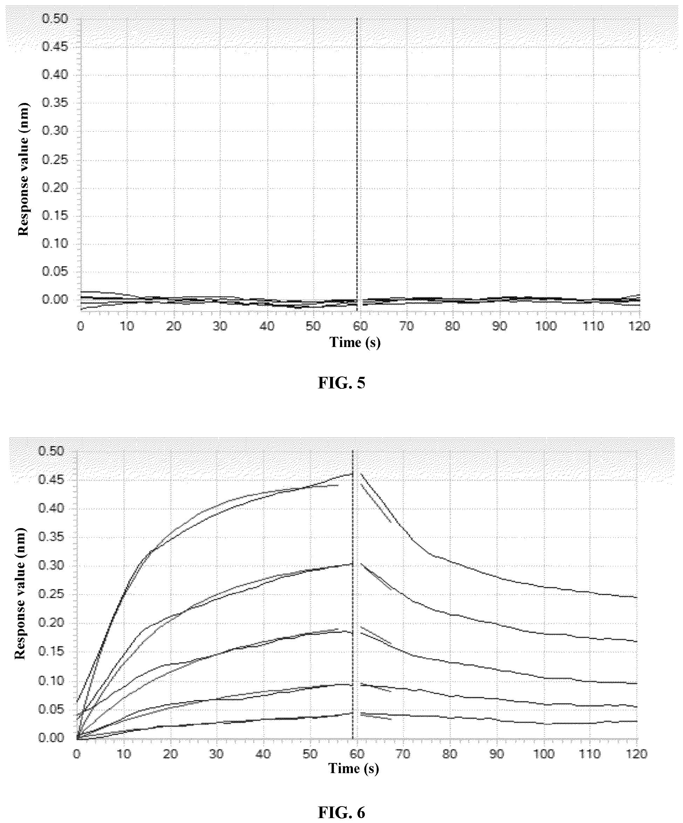

The results of affinity constant assay of BiAb004(hG1TM) and BiAb004(hG1WT) to FcγRIIIa_F158 are shown in Table 3 and FIGs. 5 and 6 below.

**Table 3: Kinetics for binding of BiAb004(hG1WT) and BiAb004(hG1TM) to FcyRIIIa F158**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.21E-07 | 1.12E+05 | 1.39E+04 | 2.47E-02 | 3.43E-03 | 0.39-0.64 |

The results show that BiAb004(hG1WT) bound to FcγRIIIa_F158 with an affinity constant of 2.21E-07 M; while for BiAb004(hG 1 TM), no corresponding data was obtained as no binding signal or an extremely weak signal to FcγRIIIa_F158 was detected, indicating no binding to FcγRIII_F158.

The results suggest that the binding activity of BiAb004(hG1TM) to FcyR IIIa_F158 is effectively eliminated as compared to BiAb004(hG 1 WT).

### Experimental Example 4: Affinity Constant Assay of BiAb004(hG1WT) and BiAb004(hG 1 TM) to FcvRIIa H131

The Fc receptor FcyRIIa_H131 (also known as CD32a_H131), can bind to the Fc fragment of IgG antibodies and mediate ADCC.

The affinity constants of BiAb004(hG 1 WT) and BiAb004(hG 1 TM) to FcyRIIa_H131 were measured in this experiment using a Fortebio Octet molecular interaction instrument to evaluate the ADCC activity of the antibodies.

The method for determining the affinity constant of BiAb004(hG 1 WT) and BiAb004(hG 1 TM) to FcyRIIa_H131 by the Fortebio Octet molecular interaction instrument is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA at pH 7.4. FcyRIIa_H131 at 5 µg/mL was immobilized on the NTA sensor at an immobilization height of about 1.0 nm. The sensor was equilibrated in a buffer of PBS, 0.02% Tween-20, 0.02% casein and 0.1% BSA at pH 7.4 for 300 s of blocking, and the binding of the immobilized FcyRIIa_H131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

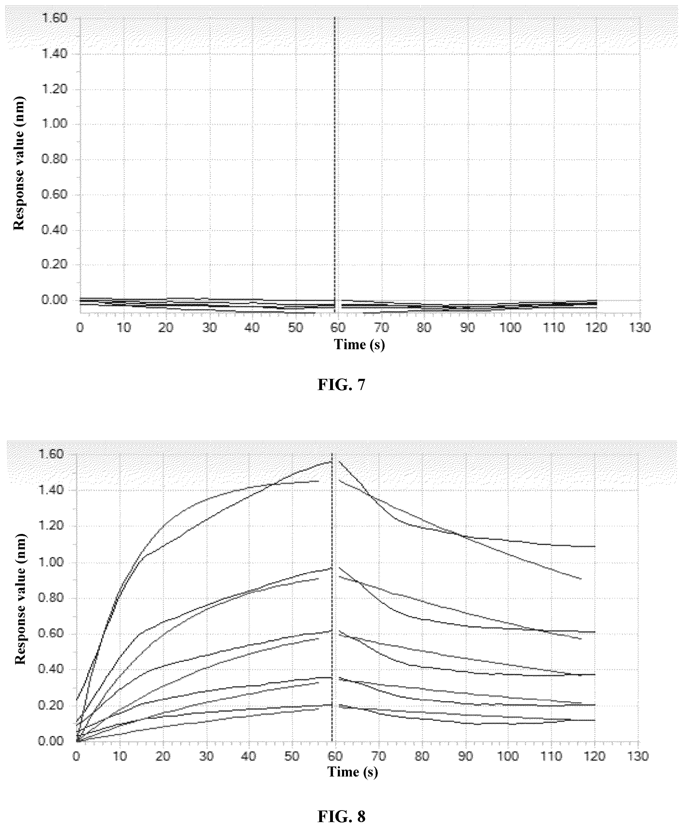

The results of affinity constant assay of BiAb004(hG1TM) and BiAb004(hG1WT) to FcyRIIa_H131 are shown in Table 4 and FIGs. 7 and 8 below.

**Table 4: Kinetics for binding of BiAb004(hG 1 WT) and BiAb004(hG 1 TM) to FcyRIIa_H131**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.22E-08 | 3.83E+05 | 4.03E+04 | 8.49E-03 | 7.44E-04 | 0.96-1.63 |

The results show that BiAb004(hG1WT) bound to FcyRIIa_H131 with an affinity constant of 2.22E-08 M; while for BiAb004(hG 1 TM), no corresponding data was obtained as no binding signal or an extremely weak signal to FcyRIIa_H131 was detected, indicating no binding to FcγRIIa_H131.

The results suggest that the binding activity of BiAb004(hG 1 TM) to FcyRIIa_H131 is effectively eliminated as compared to BiAb004(hG 1 WT).

### Experimental Example 5: Affinity Constant Assay of BiAb004(hG1WT) and BiAb004(hG 1 TM) to FcvRIIa R131

The Fc receptor FcyRIIa_R131 (also known as CD32a_R131), can bind to the Fc fragment of IgG antibodies and mediate ADCC.

The affinity constants of BiAb004(hG 1 WT) and BiAb004(hG 1 TM) to FcyRIIa_R131 were measured in this experiment using a Fortebio Octet molecular interaction instrument to evaluate the ADCC activity of the antibodies.

The method for determining the affinity constant of BiAb004(hG 1 WT) and BiAb004(hG 1 TM) by the Fortebio Octet molecular interaction instrument is briefly described as follows: the immobilization dilution buffer was a solution of PBS, 0.02% Tween-20 and 0.1% BSA at pH 7.4, and the analyte dilution buffer was a solution of PBS, 0.02% Tween-20, 0.02% casein and 0.1% BSA at pH 7.4. FcyRIIa_R131 at 5 µg/mL was immobilized on the NTA sensor at an immobilization height of about 1.0 nm. The sensor was equilibrated in a buffer of PBS, 0.02% Tween-20, 0.02% casein and 0.1% BSA at pH 7.4 for 300 s of blocking, and the binding of the immobilized FcγRIIa_R131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

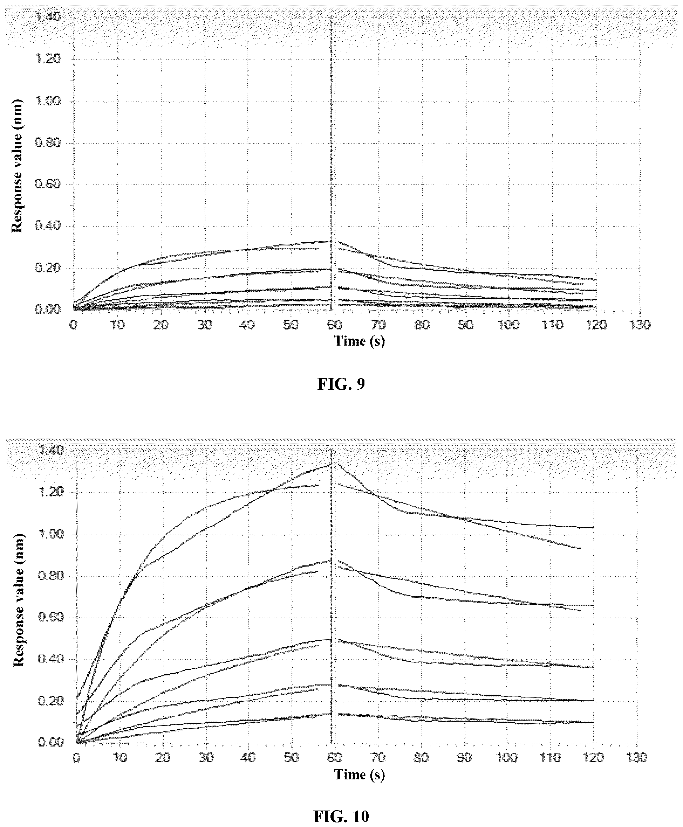

The results of affinity constant assay of **BiAb004(hG1TM)** and BiAb004(hG1WT) to FcγRIIa_R131 are shown in Table 5 and FIGs. 9 and 10 below.

**Table 5: Kinetics for binding of BiAb004(hG 1 WT) and BiAb004(hG1TM) to FcγRIIa_R131**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | 4.20E-08 | 3.72E+05 | 4.19E+04 | 1.56E-02 | 8.99E-04 | 0.16-0.36 |
| BiAb004 (hG1WT) | 1.43E-08 | 3.58E+05 | 3.13E+04 | 5.10E-03 | 5.87E-04 | 0.66-1.34 |

The results show that both BiAb004(hG1WT) and BiAb004(hG1TM) bound to FcyRIIa_R131 with affinity constants of 1.43E-08 M and 4.20E-08 M, respectively.

The results suggest that both BiAb004(hG1WT) and BiAb004(hG 1 TM) have binding activity to FcγRIIa_R131. However, the results in FIGs. 9 and 10 show that BiAb004(hG1WT) has a stronger binding signal and thus a higher affinity than that of BiAb004(hG 1 TM).

### Experimental Example 6: Affinity Constant Assay of BiAb004(hG1WT) and BiAb004(hG1TM) to FcyRIIb

The Fc receptor FcyRIIb (also known as CD32b), can bind to the Fc fragment of IgG antibodies, negatively regulate functions of immune cells, inhibit the activation and proliferation of immune cells and inhibit the secretion of cytokines.

The affinity constants of BiAb004(hG1WT) and BiAb004(hG 1 TM) to FcyRIIb were measured in this experiment using a Fortebio Octet molecular interaction instrument to evaluate binding capacity of BiAb004(hG1WT) and BiAb004(hG 1 TM) to the Fc receptor. The method for determining the affinity constant of BiAb004(hG1WT) and BiAb004(hG 1 TM) to FcyRIIb by the Fortebio Octet molecular interaction instrument is briefly described as follows: the immobilization dilution buffer was a solution of PBS, 0.02% Tween-20 and 0.1% BSA at pH 7.4, and the analyte dilution buffer was a solution of PBS, 0.02% Tween-20, 0.02% casein and 0.1% BSA at pH 7.4. FcyRIIb at 5 µg/mL was immobilized on the NTA sensor at an immobilization height of about 1.0 nm. The sensor was equilibrated in a buffer of PBS, 0.02% Tween-20, 0.02% casein and 0.1% BSA at pH 7.4 for 300 s of blocking, and the binding of the immobilized FcyRIIb on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

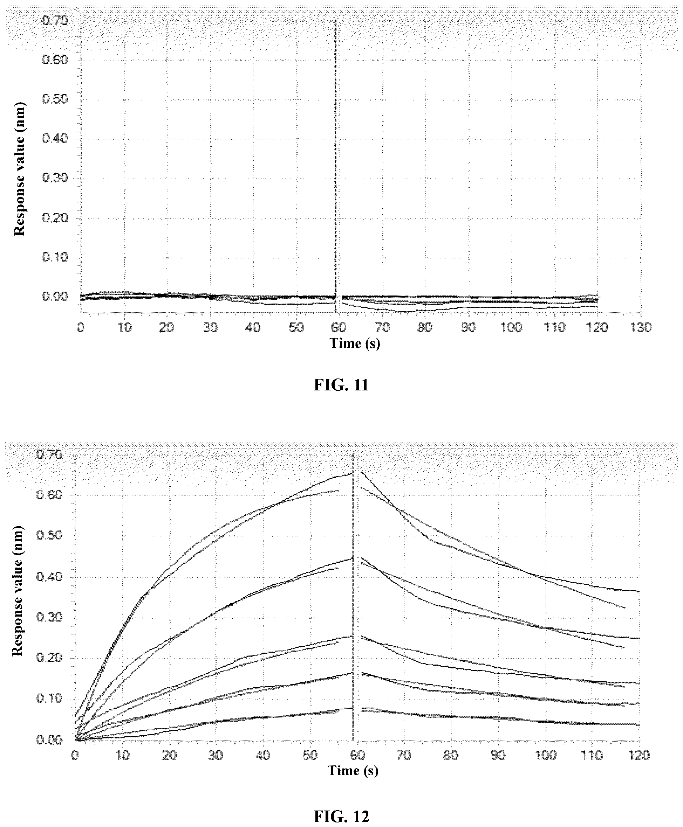

The results of affinity constant assay of BiAb004(hG1TM) and BiAb004(hG1WT) to FcyRIIb are shown in Table 6 and FIGs. 11 and 12 below.

**Table 6: Kinetics for binding of BiAb004(hG1WT) and BiAb004(hG1TM) to FcγRIIb**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 5.61E-08 | 2.07E+05 | 1.54E+04 | 1.16E-02 | 4.33E-04 | 0.68-0.83 |

The results show that BiAb004(hG1WT) bound to FcyRIIb with an affinity constant of 5.61E-08 M; while for BiAb004(hG 1 TM), no corresponding data was obtained as no binding signal or an extremely weak signal to FcyRIIb was detected, indicating no binding to FcyRIIb.

The results suggest that the binding activity of BiAb004(hG1TM) to FcyRIIb is effectively eliminated as compared to BiAb004(hG 1 WT).

### Experimental Example 7: Affinity Assay of BiAb004(hG1WT) and BiAb004(hG 1 TM) to C1q

Serum complement C1q can bind to the Fc fragment of IgG antibodies and mediate CDC effects. The binding capacity of a therapeutic monoclonal antibody to C1q will influence the safety and efficacy of the antibody.

The affinity constants of BiAb004(hG1WT) and BiAb004(hG1TM) to C1q were measured in this experiment using a Fortebio Octet molecular interaction instrument to evaluate the CDC activity of the antibodies. In this experimental example, an anti-PDL1 antibody 5C10H2L2-IgG1mt was used as a control antibody, of which the preparation method is described in PCT Publication No. WO2017148424A1.

The method for determining the affinity constants of the corresponding antibodies to C1q by the Fortebio Octet molecular interaction instrument is briefly described as follows: The sample dilution buffer was a solution of PBS, 0.02% Tween-20 and 0.1% BSA at pH 7.4. The antibody at 50 µg/mL was immobilized on the FAB2G sensor at an immobilization height of about 2.0 nm. The sensor was equilibrated in a buffer for 60 s, and the binding of the immobilized antibody on the sensor to the antigen C1q at concentrations of 1.25-20 nM (serial two-fold dilution) was determined for 60 s. The antigen-antibody was dissociated in the buffer for 60 s. The sensor was regenerated 4 times with 10 mM glycine pH 1.7, each for 5 s. The shaking speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

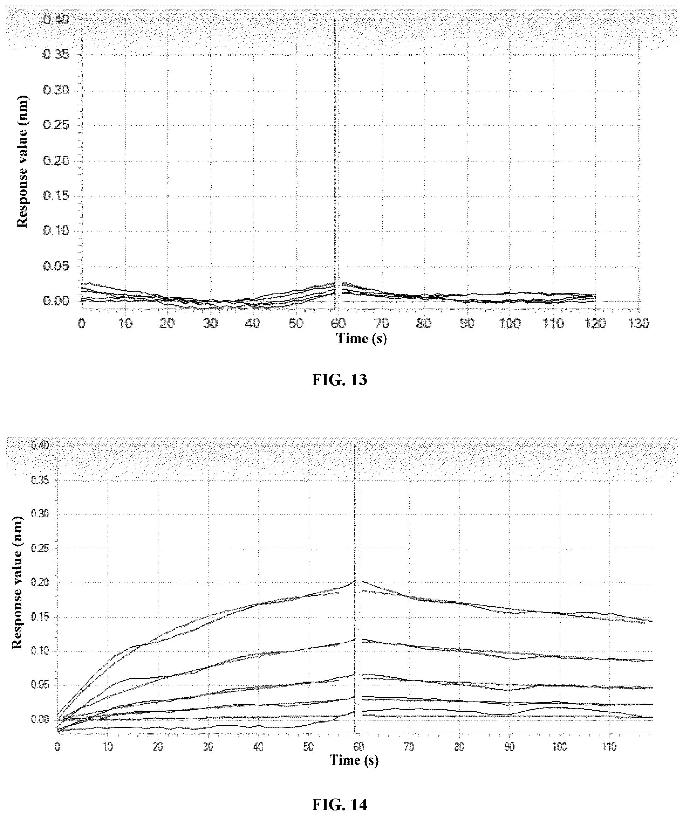

The results of affinity constant assay of BiAb004(hG1TM), BiAb004(hG1WT) and 5C10H2L2-IgG1mt to C1q are shown in Table 7 and FIGs. 13, 14 and 15 below.

**Table 7: Kinetics for binding of BiAb004(hG1WT), BiAb004(hG1TM) and 5C10H2L2-IgGlmt to C1q**

| Sample ID | K_{D} (M) | kon (1/Ms) | S E (kon) | kdis (1/s) | S E (kdis) | Rmax (nm) |
|---|---|---|---|---|---|---|
| BiAb004 (hG1TM) | N/A | N/A | N/A | N/A | N/A | N/A |
| BiAb004 (hG1WT) | 2.53E-09 | 2.05E+06 | 2.10E+05 | 5.17E-03 | 5.81E-04 | 0.05-0.23 |
| 5C10H2L2-IgG1mt | 4.43E-09 | 2.38E+06 | 4.21E+05 | 1.05E-02 | 1.10E-03 | 0.19-0.26 |

The results show that BiAb004(hG1WT) bound to C1q with an affinity constant of 2.53E-09 M; while for BiAb004(hG 1 TM), no corresponding data was obtained as no binding signal or an extremely weak signal to C1q was detected, indicating no binding to C1q.

The results suggest that the binding activity of BiAb004(hG 1 TM) to C1q is effectively eliminated as compared to BiAb004(hG 1 WT).

### Experimental Example 8; ADCC Activity Assay of BiA b004(hG 1 WT) and BiA b004(hG 1 TM) on 293T-CTLA4-PD1 Cells Expressing CTLA4 and PD-1 Antigens

The ADCC effect refers to that effector immune cells with killing activity recognize Fc fragments of antibodies bound to antigens of target cells through Fc receptors (FcR) expressed on their surfaces, and directly kill the target cells. To test the ADCC effect of the anti-CTLA4-anti-PD-1 bifunctional antibodies BiAb004(hG1WT) and BiAb004(hG1TM), the inventors constructed a co-culture system of 293T-CTLA4-PD1 cells expressing PD-1 and CTLA4 antigens and primary PBMCs to test the ADCC activity of the antibodies.

The method for ADCC activity assay of BiAb004(hG1WT) and BiAb004(hG1TM) on 293T-CTLA4-PD1 cells expressing CTLA4 and PD-1 antigens is as follows:
Normal human PBMCs were isolated according to the Ficoll peripheral blood mononuclear cell isolation instruction. The isolated PBMCs were resuspended in RPMI-1640 complete medium, stained with AO/PI, counted and frozen. One day before the experiment, PBMCs were thawed and counted, and the viability was determined. The cells were incubated overnight in a carbon dioxide incubator at 5% CO₂ and 37 °C. On the day of the experiment, 293T-CTLA4-PD1 cells and PBMCs were collected, centrifuged at 800 rpm or 1200 rpm for 5 min, resuspended in RPMI-1640 (containing 1% FBS; hereinafter referred to as the medium) and washed twice. The cells were counted and viability was determined. The cell density was adjusted to a proper range using the medium. 100 µL of 293T-CTLA4-PD1 cells was added to a 96-well plate at a density of 3.0E+04 cells/well according to the experimental design. 50 µL of antibody was added, and the cells were pre-incubated for 1 h at room temperature; after 1 h, 50 µL of PBMCs were added at 9.0E+05 cells/well and the mixture was mixed well and incubated at 37 °C for 4 h in a carbon dioxide incubator at 5% CO₂. The cells were centrifuged at 250× g for 5 min. 100 µL of the cell supernatant was transferred to a new 96-well flat-bottom microplate (do not pipette the cell precipitate). 100 µL of freshly prepared reaction solution was added to each well according to the Cytotoxicity Detection Kit instruction. The cells were incubated for 30 min at room temperature in the dark. OD values at 490 nm and 650 nm were measured. ADCC percentage was calculated for each group according to the formula ADCC(%) = (treatment group - negative control group)/(maximum LDH release in target cell - spontaneous LDH release in target cell) ×100%.

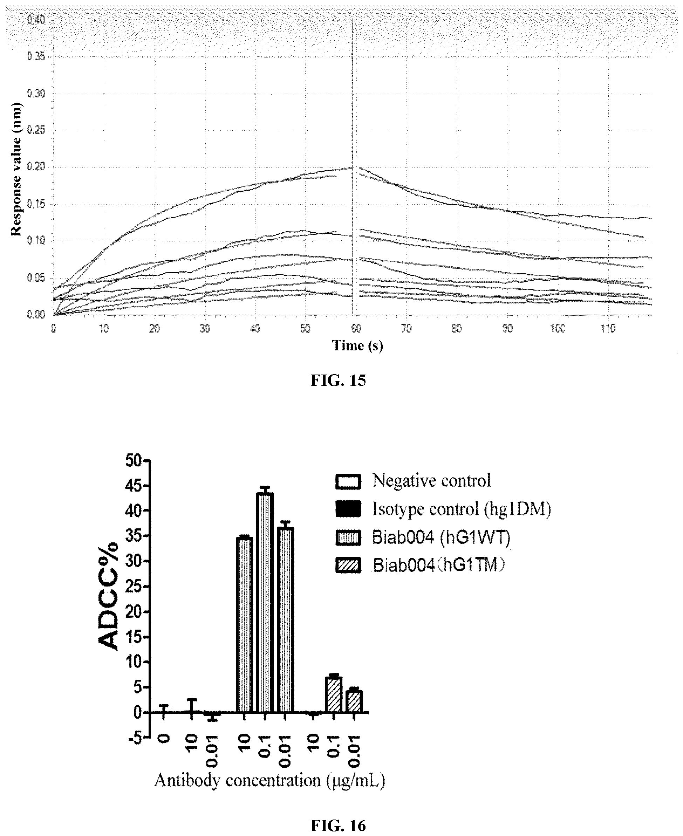

The ADCC activity of BiAb004(hG1WT) and BiAb004(hG1TM) on 293T-CTLA4-PD1 cells expressing CTLA4 and PD-1 antigens was expressed as ADCC percentages, and the results are shown in FIG. 16.

The results show that in the mixed culture system of PBMC and 293T-CTLA4-PD1, the ADCC percentage induced by BiAb004(hG 1 TM) was significantly lower than that induced by BiAb004(hG1WT) at the same dose level.

The results suggest that BiAb004(hG 1 TM) has no ADCC activity.

### Experimental Example 9: Evaluation of Activity of Anti-CTLA4/Anti-PD-1 Bispecific Antibody in Combination with Chiauranib in Lymphocyte-Tumor cell Mixed Reaction System

Freshly isolated PBMCs (from healthy donors) were adhered to the wall overnight. Mononuclear cells were collected. GM-CSF (2000 U/mL, from peprotech, Cat. No. 300-03) and IL-4 (2000 U/mL from peprotech, Cat. No. 200-04) were added. Induction was performed after 3 days of culture. For the iDC group, half of the medium was exchanged, and GM-CSF (2000 U/mL) and IL4 (2000 U/mL) were added. For the mDC group, half of the medium was exchanged, and GM-CSF (2000 U/mL), IFN-y (50 ng/mL, from Sino Biological, Cat. No. 11725-HNAS-100) and LPS (100 ng/mL, from SIGMA, Cat. No. L6529) were added. The cells were cultured in an incubator for 2 days and frozen for later use. A375 cells (purchased from ATCC, agency: Zhongyuan Ltd., Cat. No. CRL-1619) were digested conventionally, collected by centrifugation, and plated on 10 cm dishes at 2 × 10⁶ cells/dish. Chiauranib (from Chipscreen Biosciences, Lot No. 210604) was added to one of the dishes to a final concentration of 15 µM, and a blank control group was set. The mixture was incubated in an incubator at 37 °C and 5% CO₂ for 48 h (medium: DMEM + 10% FBS). PBMCs from the same donor (autologous) or a different donor (allogeneic) (from healthy donors) with iDC/mDC were thawed 1 night in advance. iDC (immature dendritic cells) and mDC (mature dendritic cells) were thawed 2h in advance. The cells were counted and plated as per DC:PBMC = 1:10 in 96-well flat-bottom plates. Untreated A375 cells and A375 cells treated with 15 µM chiauranib for 48 h were collected and added to the wells of the corresponding group at 3 × 10⁴/50 µL/well. BiAb004(hG 1 TM) was added to the wells of the corresponding group at 300 nM/50 µL/well. The mixture was incubated in an incubator for 3-5 days. The cell supernatants were separately collected on day 3 and day 5. The amounts of secretion of IFN-y (purchased from Dakewe, Cat. No. 1110202) and IL-2 (purchased from Dakewe, Cat. No. 1110202) were measured by an ELISA kit.

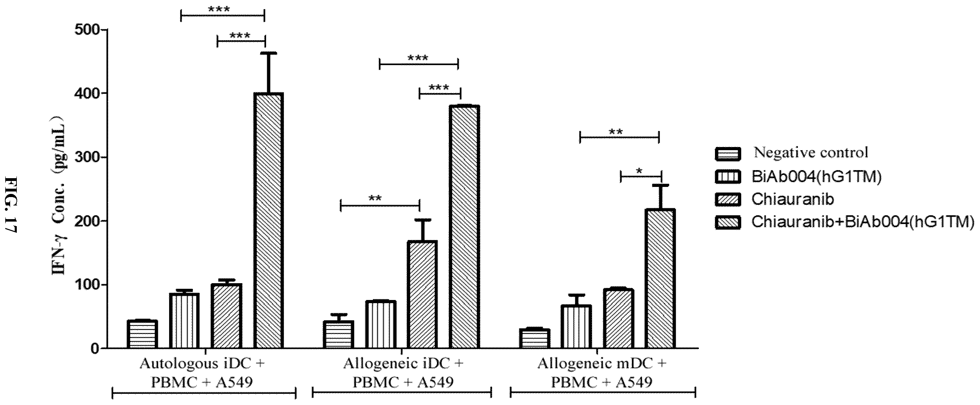

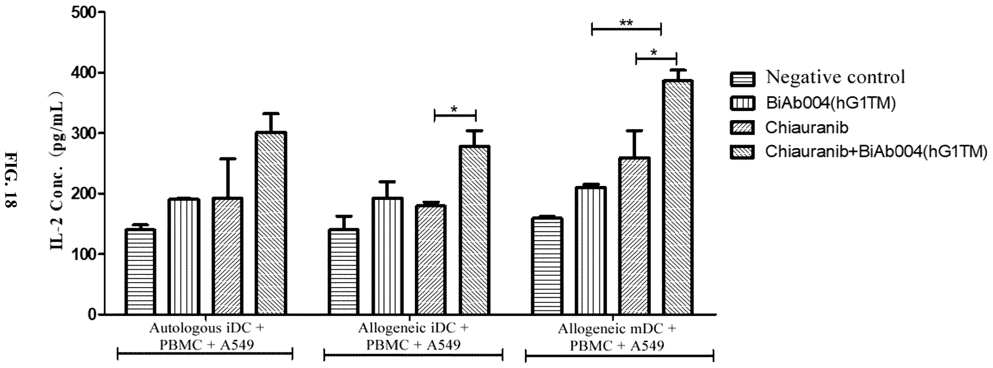

The results of assays for IFN-y secretion after mixed culture of DC, PBMC and A375 cells are shown in FIG. 17. The results of assays for IL-2 secretion after mixed culture of DC, PBMC and A375 cells are shown in FIG. 18.

As can be seen from the figures, the bifunctional antibody BiAb004(hG1TM) single-drug group, the chiauranib single-drug group and the group of the bifunctional antibody BiAb004(hG1TM) in combination with chiauranib can effectively promote the secretion of IFN-y and IL-2 in the reaction system. Among the groups, the IFN-y and IL-2 secretion promoting capability of the group of the bifunctional antibody BiAb004(hG1TM) in combination with chiauranib is separately and significantly stronger than that of the bifunctional antibody BiAb004(hG 1 TM) single-drug group and the chiauranib single-drug group.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

## Claims

1. A therapeutic combination comprising chiauranib or a pharmaceutically acceptable salt thereof (e.g., hydrochloride salt) or a crystalline form thereof (e.g., non-solvated crystalline form A, B or C) and at least one (e.g., 1, 2 or 3) anti-CTLA4-anti-PD-1 bispecific antibody.

2. The therapeutic combination according to claim 1, wherein the mass ratio of the chiauranib or the pharmaceutically acceptable salt thereof or the crystalline form thereof to the anti-CTLA4-anti-PD-1 bispecific antibody is 1:(1-1000), preferably 1:(5-500), and more preferably 1:(10-100).

3. The therapeutic combination according to any of claims 1 to 2, wherein the anti-CTLA4-anti-PD-1 bispecific antibody is in a unit dose of 100 mg-1000 mg, 200 mg-800 mg, 200 mg-500 mg, 300 mg-600 mg, 400 mg-500 mg, or 450 mg.

4. The therapeutic combination according to any of claims 1 to 3, wherein the chiauranib or the pharmaceutically acceptable salt thereof or the crystalline form thereof is in a unit dose of 0.1 mg-100 mg, 0.5 mg-50 mg, 1 mg-20 mg, 2 mg-15 mg, 3 mg-12 mg, 4 mg-8 mg, or 5 mg.

5. The therapeutic combination according to any of claims 1 to 4, wherein
the therapeutic combination is a fixed combination, e.g., a pharmaceutical composition, or the therapeutic combination is a non-fixed combination, e.g., the chiauranib or the pharmaceutically acceptable salt thereof or the crystalline form thereof and the anti-CTLA4-anti-PD-1 bispecific antibody in the non-fixed combination are each present in a separate pharmaceutical composition.

6. The therapeutic combination according to claim 5, wherein the pharmaceutical composition is independently a solid pharmaceutical composition or a liquid pharmaceutical composition.

7. The therapeutic combination according to any of claims 5 to 6, wherein the pharmaceutical composition further comprises one or more pharmaceutically acceptable adjuvants.

8. The therapeutic combination according to any of claims 5 to 7, wherein the pharmaceutical composition does not comprise an active pharmaceutical ingredient other than the chiauranib or the pharmaceutically acceptable salt thereof or the crystalline form thereof and the anti-CTLA4-anti-PD-1 bispecific antibody.

9. The therapeutic combination according to any of claims 1 to 8, wherein the anti-CTLA4-anti-PD-1 bispecific antibody comprises:
a first protein functional region targeting PD-1 and
a second protein functional region targeting CTLA4,
wherein:
the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
or
the first protein functional region is a single chain fragment variable, and the second protein functional region is an immunoglobulin, wherein a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively; a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively;
the immunoglobulin is of human IgG1 subtype,
and according to the EU numbering system, the heavy chain constant region of the immunoglobulin has mutations at any 2 or 3 of positions 234, 235 and 237, and the affinity constant of the bispecific antibody to FcyRIIIa and/or C1q is reduced after the mutation as compared to that before the mutation; preferably, the affinity constant is measured by a Fortebio Octet molecular interaction instrument.

10. The therapeutic combination according to any of claims 1 to 9, wherein the anti-CTLA4-anti-PD-1 bispecific antibody is provided, wherein according to the EU numbering system, the heavy chain constant region of the immunoglobulin has the following mutations:
L234A and L235A; or
L234A and G237A; or
L235A and G237A;
or
L234A, L235A and G237A.

11. The therapeutic combination according to any of claims 1 to 10, wherein the anti-CTLA4-anti-PD-1 bispecific antibody comprises:
a first protein functional region targeting PD-1 and
a second protein functional region targeting CTLA4,
wherein:
the first protein functional region is an immunoglobulin, and the second protein functional region is a single chain fragment variable, wherein a heavy chain variable region of the immunoglobulin comprises HCDR1-HCDR3 having amino acid sequences set forth in SEQ ID NOs: 27-29, respectively, and a light chain variable region of the immunoglobulin comprises LCDR1-LCDR3 having amino acid sequences set forth in SEQ ID NOs: 30-32, respectively; a heavy chain variable region of the single chain fragment variable comprises HCDR1-HCDR3 having amino acid sequences set forth in SEQ ID NOs: 33-35, respectively, and a light chain variable region of the single chain fragment variable comprises LCDR1-LCDR3 having amino acid sequences set forth in SEQ ID NOs: 36-38, respectively;
or
the first protein functional region is a single chain fragment variable, and the second protein functional region is an immunoglobulin, wherein a heavy chain variable region of the immunoglobulin comprises HCDR1-HCDR3 having amino acid sequences set forth in SEQ ID NOs: 33-35, respectively, and a light chain variable region of the immunoglobulin comprises LCDR1-LCDR3 having amino acid sequences set forth in SEQ ID NOs: 36-38, respectively; a heavy chain variable region of the single chain fragment variable comprises HCDR1-HCDR3 having amino acid sequences set forth in SEQ ID NOs: 27-29, respectively, and a light chain variable region of the single chain fragment variable comprises LCDR1-LCDR3 having amino acid sequences set forth in SEQ ID NOs: 30-32, respectively;
the immunoglobulin is of human IgG1 subtype,
according to the EU numbering system, the immunoglobulin comprises a heavy chain constant region having one of the following mutations:
L234A and L235A; or
L234A and G237A; or
L235A and G237A; or
L234A, L235A and G237A.

12. The therapeutic combination according to any of claims 1 to 11, wherein the anti-CTLA4-anti-PD-1 bispecific antibody is provided, wherein according to the EU numbering system, the heavy chain constant region of the immunoglobulin further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

13. The therapeutic combination according to any of claims 1 to 12, wherein the anti-CTLA4-anti-PD-1 bispecific antibody is provided, wherein
an amino acid sequence of the heavy chain variable region of the immunoglobulin is selected from SEQ ID NO: 14 and SEQ ID NO: 18, and an amino acid sequence of the light chain variable region of the immunoglobulin is selected from SEQ ID NO: 16 and SEQ ID NO: 20; an amino acid sequence of the heavy chain variable region of the single chain fragment variable is selected from SEQ ID NO: 2, SEQ ID NO: 6, SEQ ID NO: 10, SEQ ID NO: 41 and SEQ ID NO: 43, and an amino acid sequence of the light chain variable region of the single chain fragment variable is selected from SEQ ID NO: 4, SEQ ID NO: 8, SEQ ID NO: 12, SEQ ID NO: 42 and SEQ ID NO: 44;
or
an amino acid sequence of the heavy chain variable region of the immunoglobulin is selected from SEQ ID NO: 2, SEQ ID NO: 6, SEQ ID NO: 10, SEQ ID NO: 41 and SEQ ID NO: 43, and an amino acid sequence of the light chain variable region of the immunoglobulin is selected from SEQ ID NO: 4, SEQ ID NO: 8, SEQ ID NO: 12, SEQ ID NO: 42 and SEQ ID NO: 44; an amino acid sequence of the heavy chain variable region of the single chain fragment variable is selected from SEQ ID NO: 14 and SEQ ID NO: 18, and an amino acid sequence of the light chain variable region of the single chain fragment variable is selected from SEQ ID NO: 16 and SEQ ID NO: 20.

14. The therapeutic combination according to any of claims 1 to 13, wherein the anti-CTLA4-anti-PD-1 bispecific antibody is selected from any one of the following (1)-(20):
(1)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(2)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(3)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(4)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 2, and an amino acid sequence of the light chain variable region of the single chain fragment variable is set forth in SEQ ID NO: 4;
(5)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 6, and an amino acid sequence of the light chain variable region of the single chain fragment variable is set forth in SEQ ID NO: 8;
(6)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 10, and an amino acid sequence of the light chain variable region of the single chain fragment variable is set forth in SEQ ID NO: 12;
(7)
an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(8)
an amino acid sequence of the heavy chain variable region of the immunoglobulin is set forth in SEQ ID NO: 2, and an amino acid sequence of the light chain variable region of the immunoglobulin is set forth in SEQ ID NO: 4; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(9)
an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(10)
an amino acid sequence of the heavy chain variable region of the immunoglobulin is set forth in SEQ ID NO: 6, and an amino acid sequence of the light chain variable region of the immunoglobulin is set forth in SEQ ID NO: 8; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(11)
an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(12)
an amino acid sequence of the heavy chain variable region of the immunoglobulin is set forth in SEQ ID NO: 10, and an amino acid sequence of the light chain variable region of the immunoglobulin is set forth in SEQ ID NO: 12; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
(13)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(14)
an amino acid sequence of the heavy chain variable region of the immunoglobulin is set forth in SEQ ID NO: 14, and an amino acid sequence of the light chain variable region of the immunoglobulin is set forth in SEQ ID NO: 16; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(15)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 41, and an amino acid sequence of the light chain variable region of the single chain fragment variable is set forth in SEQ ID NO: 42;
(16)
an amino acid sequence of the heavy chain variable region of the immunoglobulin is set forth in SEQ ID NO: 18, and an amino acid sequence of the light chain variable region of the immunoglobulin is set forth in SEQ ID NO: 20; an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
(17)
an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(18)
an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 14, and an amino acid sequence of the light chain variable region of the single chain fragment variable is set forth in SEQ ID NO: 16;
(19)
an amino acid sequence of the heavy chain variable region of the immunoglobulin is set forth in SEQ ID NO: 41, and an amino acid sequence of the light chain variable region of the immunoglobulin is set forth in SEQ ID NO: 42; an amino acid sequence of the heavy chain variable region of the single chain fragment variable is set forth in SEQ ID NO: 18, and an amino acid sequence of the light chain variable region of the single chain fragment variable is set forth in SEQ ID NO: 20;
and
(20)
an amino acid sequence of the heavy chain variable region of the immunoglobulin is set forth in SEQ ID NO: 43, and an amino acid sequence of the light chain variable region of the immunoglobulin is set forth in SEQ ID NO: 44; an amino acid sequence of the heavy chain variable region of the single chain fragment variable is set forth in SEQ ID NO: 18, and an amino acid sequence of the light chain variable region of the single chain fragment variable is set forth in SEQ ID NO: 20.

15. The therapeutic combination according to any of claims 1 to 14, wherein the anti-CTLA4-anti-PD-1 bispecific antibody is provided:
an amino acid sequence of the heavy chain of the immunoglobulin is set forth in SEQ ID NO: 40, and an amino acid sequence of the light chain is set forth in SEQ ID NO: 24.

16. The therapeutic combination according to any of claims 1 to 15, wherein the anti-CTLA4-anti-PD-1 bispecific antibody is provided, wherein the immunoglobulin or the antigen-binding fragment thereof binds to FcγRIIIa_F158, FcyRI, FcyRIIa_H131, FcγRIIIa_V158 and/or FcyRIIb with an affinity constant greater than about 10⁻⁷ M, for example, greater than about 10⁻⁶ M, 10⁻⁵ M, 10⁻⁴ M, or 10⁻³ M or greater; preferably, the affinity constant is measured by a Fortebio Octet molecular interaction instrument;
preferably, the immunoglobulin or the antigen-binding fragment thereof has no binding signal or a binding signal of less than 0.1 nm to FcγRIIIa_F158, FcyRI, FcyRIIa_H131, FcγRIIIa_V158 and/or FcyRIIb; preferably, the binding signal refers to a response value measured by a Fortebio Octet molecular interaction instrument.

17. The therapeutic combination according to any of claims 1 to 16, wherein the anti-CTLA4-anti-PD-1 bispecific antibody is provided, wherein the immunoglobulin or the antigen-binding fragment thereof binds to C1q with an affinity constant greater than about 10⁻⁹ M, for example, greater than about 10⁻⁸ M, 10⁻⁷ M, 10⁻⁶ M, or 10⁻⁵ M or greater; preferably, the affinity constant is measured by a Fortebio Octet molecular interaction instrument; preferably, the immunoglobulin or the antigen-binding fragment thereof has no binding signal or a binding signal of less than 0.1 nm to C1q; preferably, the binding signal refers to a response value measured by a Fortebio Octet molecular interaction instrument.

18. The therapeutic combination according to any of claims 1 to 17, wherein the anti-CTLA4-anti-PD-1 bispecific antibody is provided, wherein the first protein functional region is linked to the second protein functional region either directly or via a linker fragment, and/or the heavy chain variable region of the single chain fragment variable is linked to the light chain variable region of the single chain fragment variable either directly or via a linker fragment.

19. The therapeutic combination according to any of claims 1 to 18, wherein the anti-CTLA4-anti-PD-1 bispecific antibody is provided, wherein the linker fragment is (GGGGS)n, n being a positive integer; preferably, n is 1, 2, 3, 4, 5 or 6.

20. The therapeutic combination according to any of claims 1 to 19, wherein the anti-CTLA4-anti-PD-1 bispecific antibody is provided, wherein the numbers of the first protein functional region and the second protein functional region are each independently 1, 2, or more.

21. The therapeutic combination according to any of claims 1 to 20, wherein the anti-CTLA4-anti-PD-1 bispecific antibody is provided, wherein the single chain fragment variable is linked to the C terminus of the heavy chain of the immunoglobulin.

22. The therapeutic combination according to any of claims 1 to 21, wherein the anti-CTLA4-anti-PD-1 bispecific antibody comprises:
a first protein functional region targeting PD-1 and
a second protein functional region targeting CTLA4,
the number of the first protein functional region is 1, and the number of the second protein functional region is 2;
wherein the first protein functional region is an immunoglobulin, and the second protein functional region is a single chain fragment variable;
an amino acid sequence of the heavy chain of the immunoglobulin is set forth in SEQ ID NO: 40, and an amino acid sequence of the light chain is set forth in SEQ ID NO: 24;
an amino acid sequence of the heavy chain variable region of the single chain fragment variable is set forth in SEQ ID NO: 43, and an amino acid sequence of the light chain variable region of the single chain fragment variable is set forth in SEQ ID NO: 44;
the single chain fragment variable is linked to the C terminus of the heavy chain of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NO: 25 and SEQ ID NO: 26;
preferably, amino acid sequences of the first linker fragment and the second linker fragment are set forth in SEQ ID NO: 26.

23. A kit product comprising the therapeutic combination according to any of claims 1 to 22, and a package insert.

24. Use of the therapeutic combination according to any of claims 1 to 22 for preparing a drug for the treatment or prevention of a tumor;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

25. The therapeutic combination according to any of claims 1 to 22 for use in the treatment or prevention of a tumor;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

26. A method for treating or preventing a tumor, comprising a step of administering to a subject in need an effective amount of the therapeutic combination according to any of claims 1 to 22;
preferably, the tumor is selected from one or more of melanoma, renal cancer, prostate cancer, bladder cancer, colon cancer, rectal cancer, gastric cancer, liver cancer, lung cancer, ovarian cancer, leukemia, breast cancer, mesothelioma, cervical cancer, endometrial cancer, lymphoma, pancreatic cancer, and nasopharyngeal cancer;
preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.
